(19)

Europäisches
Patentamt

European
Patent Office

Office européen
des brevets

(11) **EP 3 632 988 A1**

(12) **EUROPEAN PATENT APPLICATION**
published in accordance with Art. 153(4) EPC

(43) Date of publication:
**08.04.2020 Bulletin 2020/15**

(21) Application number: **18809406.4**

(22) Date of filing: **31.05.2018**

(51) Int Cl.:
*C08L 89/00* (2006.01)          *C08K 5/529* (2006.01)
*D01F 4/02* (2006.01)          *C12N 15/00* (2006.01)

(86) International application number:
**PCT/JP2018/021051**

(87) International publication number:
**WO 2018/221680 (06.12.2018 Gazette 2018/49)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **02.06.2017   JP 2017109974**

(71) Applicants:
• **Institute of National Colleges of Technology,
Japan
Hachioji-shi, Tokyo 193-0834 (JP)**
• **Spiber Inc.
Yamagata 997-0052 (JP)**

(72) Inventors:
• **SATO Takaya
Tsuruoka-shi
Yamagata 997-8511 (JP)**

• **MORINAGA Takashi
Tsuruoka-shi
Yamagata 997-8511 (JP)**
• **SATOH Ryo
Tsuruoka-shi
Yamagata 997-8511 (JP)**
• **TAKAHASHI Kentaro
Tsuruoka-shi
Yamagata 997-0052 (JP)**
• **SATO Takehiro
Tsuruoka-shi
Yamagata 997-0052 (JP)**

(74) Representative: **Handley, Matthew Edward
Venner Shipley LLP
200 Aldersgate
London EC1A 4HD (GB)**

(54) **PROTEIN MOLDED ARTICLE AND METHOD FOR PRODUCING SAME, PROTEIN SOLUTION,
AND PROTEIN MOLDED ARTICLE PLASTICIZER**

(57)    The present invention relates to a protein molded article including a protein, in which an ionic liquid is included inside the protein molded article. The present invention also relates to a production method of the protein molded article, including a step of adding the ionic liquid to a molding material of the protein molded article. Further, the present invention relates to a protein solution including a protein, an ionic liquid, and a solvent. Further, the present invention also relates to a protein molded article plasticizer including an ionic liquid.

**EP 3 632 988 A1**

## Description

## Technical Field

The present invention relates to a protein molded article and a method for producing the same, a protein solution, and a protein molded article plasticizer.

## Background Art

Due to the rise in awareness of environment preservation, consideration of alternative materials for materials derived from petroleum has been promoted. Structural proteins, which are excellent in twits of strength etc., are considered as candidates for such alternative materials. For example, molded articles such as cast films or fibers formed of structural proteins have been proposed.
It is disclosed that, as a method for improving flexibility and strength of films and fibers made of a structural protein, silk fibroin, a plasticizer such as glycerol is added to the molded article (Patent Literature 1).

## Citation List

## Patent Literature

Patent Literature 1: WO 2005/103158 A

## Summary of Invention

## Problems to be Solved by the Invention

An organic solvent such as glycerol used as a plasticizer in Patent Literature 1 is volatile, thus making it difficult to prevent gradual deterioration of flexibility and elongation of the molded article including such a plasticizer, as the plasticizer volatilizes and escapes over time. Thus, an alternative method for improving flexibility and elongation of the protein molded article is needed.
An object of the present invention is to provide a protein molded article improved in flexibility and elongation and a method for producing the protein molded article. Further, another object of the present invention is to provide a protein solution used for producing the protein molded article. Further, still another object of the present invention is to provide a protein molded article plasticizer capable of conferring excellent flexibility and elongation to the protein molded article.

## Means for Solving the Problems

The present invention relates to each of the following inventions, for example.

[1] A protein molded article including a protein, in which
an ionic liquid is included inside the protein molded article.
[2] The protein molded article according to [1], in which the ionic liquid is dispersed inside the protein molded article.
[3] The protein molded article according to [1] or [2], in which the protein is a structural protein.
[4] The protein molded article according to [3], in which the structural protein is fibroin.
[5] The protein molded article according to [4], in which the fibroin is spider silk fibroin.
[6] The protein molded article according to any one of [1] to [5], in which the ionic liquid is a hydrophobic ionic liquid.
[7] The protein molded article according to any one of [1] to [6], in which the protein molded article is a fiber or a film.
[8] The protein molded article according to any one of [1] to [7], in which a temperature at which a heat weight loss ratio of the ionic liquid is 10% by mass is 200°C or higher.
[9] A production method of the protein molded article according to any one of [1] to [8], including a step of adding the ionic liquid to a molding material of the protein molded article.
[10] The production method according to [9], in which the step of adding the ionic liquid is a step of causing infiltration of the ionic liquid into the molding material of the protein molded article.
[11] The production method according to [9], in which the step of adding the ionic liquid is a step of mixing the molding material of the protein molded article and the ionic liquid.
[12] The production method according to [11], in which the molding material is a molding solution prepared by dissolving the protein in a solvent.

[13] The production method according to [12], further including a step of obtaining the protein molded article by coagulating the molding solution to which the ionic liquid is mixed through contact with a coagulating liquid,
in which the coagulating liquid includes an ion-supplying substance for supplying an ion to the coagulating liquid.
[14] The production method according to [13], in which the ion-supplying substance is a substance capable of causing coagulation of the protein.
[15] The production method of the protein molded article according to any one of [1] to [8], including a step of obtaining a molded article by molding the molding material of the protein molded article and then causing infiltration of the ionic liquid into the molded article.
[16] A protein solution including a protein, an ionic liquid, and a solvent.
[17] The protein solution according to [16], in which a content of the ionic liquid is from 10 to 20% by mass on a basis of a whole amount of the protein solution.
[18] The protein solution according to [16] or [17], in which the protein is a structural protein.
[19] The protein solution according to [18], in which the structural protein is fibroin.
[20] The protein solution according to [19], in which the fibroin is spider silk fibroin.
[21] A protein molded article plasticizer including an ionic liquid.
[22] The protein molded article plasticizer according to [21], in which the protein is a structural protein.
[23] The protein molded article plasticizer according to [22], in which the structural protein is fibroin.
[24] The protein molded article plasticizer according to [23], in which the fibroin is spider silk fibroin.

**Effects of the Invention**

According to the present invention, there is provided an object of the present invention is to provide a protein molded article improved in flexibility and elongation and a method for producing the protein molded article. According to the present invention, there is also provided a protein solution used for producing the protein molded article. According to the present invention, there is further provided a protein molded article plasticizer capable of conferring excellent flexibility and elongation to the protein molded article. Further, the protein molded article according to the present embodiment can prevent a reduction in stress to some extent, thereby showing improvement in toughness in addition to flexibility and elongation.

**Brief Description of Drawings**

FIG. 1 is a schematic view showing an example of a spinning device for producing protein raw material fibers.
FIG. 2(A) shows an energy dispersive X-ray analysis (EDX) spectrum of a cross section of protein fibers of Example 1. FIG. 2(B) shows an EDX spectrum of a cross section of protein fibers of Example 2.
FIG. 3 is a graph showing a proportion of phosphorus with respect to carbon in the cross section of the protein fibers of Example 1 and Example 2.
FIG. 4 is a graph showing the results of the tensile test of the protein fibers of Example 1 and Example 2.
FIG. 5(A) shows an EDX spectrum of a cross section of a protein film of Test Example 2. FIG. 5(B) is a diagram showing results of surface analysis according to EDX of the cross section of the protein film of Test Example 2.
FIG. 6 is an image showing results obtained by observing the solubility of the spider silk fibroins of Example 3.
FIG. 7 is an image showing results obtained by observing the solubility of the spider silk fibroins of Example 3.
FIG. 8 is an image showing a scanning electron microscope image of a protein film A of Test Example 4. FIG. 8(A) is an image obtained by imaging the surface of the protein film A. FIG. 8(B) is an image obtained by imaging an end (edge) of the protein film A.
FIG. 9(A) shows an EDX spectrum of a cross section of a protein film A of Test Example 4. FIG. 9(B) is a diagram showing results of surface analysis according to EDX of the cross section of the protein film of Test Example 4.
FIG. 10 is a graph showing the results of the tensile test of the protein film of Test Example 4. FIG. 10(A) is a graph showing the results of the tensile test of the protein film A (ionic liquid included). FIG. 10(B) is a graph showing the results of the tensile test of the protein film B (ionic liquid not included).

**Embodiments for Carrying Out the Invention**

Embodiments of the present invention are described in detail below. However, the present invention is not limited to the following Embodiments.

[Protein molded article]

The protein molded article according to the present embodiment includes a protein, and an ionic liquid is included inside the protein molded article. The protein molded article according to the present embodiment improves in flexibility and elongation by including the ionic liquid in its inside. Further, the protein molded article according to the present embodiment can prevent a reduction in stress to some extent, thereby showing improvement in toughness in addition to flexibility and elongation. In the protein molded article according to the present embodiment, the ionic liquid is preferably dispersed inside the protein molded article for more remarkably exhibiting the advantageous effects of the present invention.

(Protein)

The protein molded article according to the present embodiment includes a protein as a main component. The protein is not particularly limited, and it may be produced in a microorganism or the like by a gene recombination technique, produced by synthesis, or produced by purifying a naturally derived protein.
The above-mentioned protein may be, for example, a structural protein and an artificial structural protein derived from the structural protein. The structural protein refers to a protein that forms or maintains a structure, a morphology, and the like in vivo. Examples of the structural protein include fibroin, keratin, collagen, elastin, and resilin.
The structural protein may be fibroin. Fibroin may be, for example, one or more members selected from the group consisting of silk fibroin, spider silk fibroin, and hornet silk fibroin. In particular, the structural protein may be silk fibroin, spider silk fibroin, or a combination thereof. When silk fibroin and spider silk fibroin are used in combination, the ratio of silk fibroin may be, for example, 40 parts by mass or less, 30 parts by mass or less, or 10 parts by mass or less, based on 100 parts by mass of spider silk fibroin.
The silk fibroin may be sericin-removed silk fibroin, sericin-unremoved silk fibroin, or a combination thereof. Sericin-removed silk fibroin is obtained by purifying silk fibroin by removing sericin covering the silk fibroin, other fats, etc. The silk fibroin purified in this manner is preferably used as a freeze-dried powder. Sericin-unremoved silk fibroin is unpurified silk fibroin from which sericin etc. are not removed.
Spider silk fibroin may also contain a spider silk polypeptide selected from the group consisting of natural spider silk proteins and polypeptides derived from natural spider silk proteins (artificial spider silk protein).
Examples of natural spider silk proteins include spigot dragline proteins, spiral line proteins, and minor ampullate gland proteins. The spigot dragline has a repetitive region composed of crystalline and amorphous regions, and has high stress and stretchability. The spider spiral line does not have crystalline regions, but have a repetitive region composed of amorphous regions. The spiral line has high stretchability, although its stress is inferior to that of the spigot dragline.
Spigot dragline proteins are produced in the major ampullate glands of spiders, and characteristically have excellent toughness. Examples of Spigot dragline proteins include major ampullate spidroins MaSp1 and MaSp2 derived from Nephila clavipes, and ADF3 and ADF4 derived from Araneus diadematus. ADF3 is one of the two primary dragline proteins of Araneus diadematus. Polypeptides derived from natural spider silk proteins may be polypeptides derived from these dragline proteins. Polypeptides derived from ADF3 can be relatively easily synthesized, and have excellent characteristics in terms of high elongation and toughness.
Spiral line proteins are produced in the flagelliform glands of spiders. Examples of spiral line proteins include flagelliform silk proteins derived from Nephila clavipes.
Polypeptides derived from natural spider silk proteins may be recombinant spider silk proteins. Examples of recombinant spider silk proteins include variants, analogs, derivatives, or the like of natural spider silk proteins. Preferable examples of such polypeptides include recombinant spider silk proteins of spigot dragline proteins (hereinafter also referred to as "polypeptides derived from spigot dragline proteins).
Examples of the proteins derived from the spigot dragline and the proteins derived from silkworm silk, both of which are fibroin-like proteins, include proteins containing a domain sequence represented by the formula 1: $[(A)_n$ motif-$REP]_m$ or the formula 2: $[(A)_n$ motif-$REP]_m$ - $(A)_n$ motif. In these formulae, the $(A)_n$ motif represents an amino acid sequence mainly composed of alanine residues, and the number of amino acid residues is from 2 to 27. The number of amino acid residues of the $(A)_n$ motif may be an integer of 2 to 20, 4 to 27, 4 to 20, 8 to 20, 10 to 20, 4 to 16, 8 to 16, or 10 to 16. Further, a ratio of the number of alanine residues with respect to the total number of amino acid residues in the $(A)_n$ motif only needs to be 40% or more, and the ratio may be 60% or more, 70% or more, 80% or more, 83% or more, 85% or more, 86% or more, 90% or more, 95% or more, or 100% (meaning that the motif is composed of only alanine residues). Among a plurality of the $(A)_n$ motifs present in the domain sequence, at least seven motifs may be composed of only alanine residues. REP represents an amino acid sequence composed of 2 to 200 amino acid residues. REP may be an amino acid sequence composed of 10 to 200 amino acid residues. In the formulae, m represents an integer of 2 to 300, and m may be an integer of 10 to 300. The plurality of the $(A)_n$ motifs may have the same or different amino acid sequences. A plurality of REPs may have the same or different amino acid sequences. Specific examples of the proteins derived from the spigot dragline include proteins having amino acid sequences represented by SEQ ID NO: 1 and SEQ ID NO: 8.

Examples of proteins derived from spiral line proteins include proteins containing a domain sequence represented by the formula 3: $[REP2]_o$ (here, in the formula 3, REP2 represents an amino acid sequence composed of Gly-Pro-Gly-Gly-X; X represents at least one amino acid selected from the group consisting of alanine (Ala), serine (Ser), tyrosine (Tyr), and valine (Val); and o represents an integer of 8 to 300). Specific examples thereof include proteins including the amino acid sequence represented by SEQ ID NO: 2. The amino acid sequence represented by SEQ ID NO: 2 is obtained by bonding an amino acid sequence (referred to as the PR1 sequence) from the 1220th residue to the 1659th residue from the N-terminal corresponding to the repeated part and motif of a partial sequence of flagelliform silk protein of Nephila clavipes obtained from the NCBI database (NCBI Accession Number: AAF36090, GI: 7106224) to a C-terminal amino-acid sequence from the 816th residue to the 907th residue from the C-terminal of a partial sequence of flagelliform silk protein of Nephila clavipes obtained from the NCBI database (NCBI Accession Number: AAC38847, GI: 2833649); and adding the amino acid sequence represented by SEQ ID NO: 7 (tag sequence and hinge sequence) to the N-terminal of the bound sequence.

Examples of proteins derived from collagen include proteins containing a domain sequence represented by the formula 4: $[REP3]_p$ (here, in the formula 4, p represents an integer of 5 to 300; REP3 represents an amino acid sequence composed of Gly-X-Y; X and Y represent any amino acid residues other than Gly; and a plurality of REP3 may be the same or different amino acid sequences). Specific examples thereof include proteins including the amino acid sequence represented by SEQ ID NO: 3. The amino acid sequence represented by SEQ ID NO: 3 is obtained by adding the amino acid sequence represented by SEQ ID NO: 7 (tag sequence and hinge sequence) to the N-terminal of an amino acid sequence from the 301st residue to the 540th residue corresponding to the repeated part and motif of a partial sequence of human collagen type 4 obtained from the NCBI database (NCBI GenBank Accession Number: CAA56335.1, GI: 3702452).

Examples of proteins derived from resilin include proteins containing a domain sequence represented by the formula 5: $[REP4]_q$ (here, in the formula 5, q represents an integer of 4 to 300; REP4 represents an amino acid sequence composed of Ser-J-J-Tyr-Gly-U-Pro; J represents any amino acid residue, and particularly preferably an amino acid residue selected from the group consisting of Asp, Ser, and Thr; U represents any amino acid residue, and particularly preferably an amino acid residue selected from the group consisting of Pro, Ala, Thr, and Ser; and a plurality of REP4 may be the same or different amino acid sequences). Specific examples thereof include proteins including the amino acid sequence represented by SEQ ID NO: 4. The amino acid sequence represented by SEQ ID NO: 4 is obtained by adding the amino acid sequence represented by SEQ ID NO: 7 (tag sequence and hinge sequence) to the N-terminal of an amino acid sequence from the 19th residue to the 321st residue of a sequence obtained by substituting the 87th residue Thr with Ser, and also substituting the 95th residue Asn with Asp in the amino acid sequence of resilin (NCBI GenBank Accession Number: NP 611157, G1: 24654243).

Examples of proteins derived from elastin include proteins having amino acid sequences such as those of NCBI GenBank Accession Numbers: AAC98395 (human), 147076 (sheep), and NP786966 (cow). Specific examples thereof include proteins including the amino acid sequence represented by SEQ ID NO: 5. The amino acid sequence represented by SEQ ID NO: 5 is obtained by adding the amino acid sequence represented by SEQ ID NO: 7 (tag sequence and hinge sequence) to the N-terminal of an amino acid sequence from the 121st residue to the 390th residue of the amino acid sequence of NCBI GenBank Accession Number: AAC98395.

Examples of proteins derived from keratin include type I keratin of Capra hircus, etc. Specific examples thereof include proteins including the amino acid sequence represented by SEQ ID NO: 6 (amino acid sequence of NCBI GenBank Accession Number: ACY30466).

The abovementioned structural proteins and proteins derived from the structural proteins can be used singly or in combination of two or more.


(Production method of proteins)

The protein described above can be produced by, for example, expressing a nucleic acid encoding the protein using a host transformed with an expression vector having one or more regulatory sequences operably linked to the sequence of the nucleic acid.

The method for producing the nucleic acid encoding the protein is not particularly limited. For example, the nucleic acid can be produced by a method of amplifying a gene by polymerase chain reaction (PCR) etc. for cloning, or by a chemical synthesis method, both using a gene encoding a natural structural protein. The method for chemically synthesizing the nucleic acid is also not particularly limited. For example, a gene can be chemically synthesized by linking oligonucleotides automatically synthesized using AKTA oligopilot plus 10/100 (commercially available from GE Healthcare Japan), etc., by PCR or the like based on amino acid sequence information of structural proteins obtained from the NCBI web database, etc. Under this circumstance, in order to facilitate the purification and/or confirmation of the protein, it is possible to synthesize a nucleic acid encoding a protein including an amino acid sequence obtained by adding an amino acid sequence composed of a start codon and His 10 tags to the N-terminal of the abovementioned amino acid sequence.

The regulatory sequence is a sequence that regulates the expression of a recombinant protein in a host (e.g., a promoter, an enhancer, a ribosome-binding sequence, a transcriptional termination sequence, etc.). The regulatory sequence can be suitably selected depending on the type of host. The promoter may be an inducible promoter that functions in host cells, and can induce the expression of a target protein. The inducible promoter is a promoter that can control transfer by the presence of an inductor (an expression-inducing agent), the absence of repressor molecules, or physical factors such as increase or decrease in temperature, osmotic pressure, or pH value.

The type of expression vector can be suitably selected from plasmid vectors, viral vectors, cosmid vectors, fosmid vectors, artificial chromosome vectors, etc., depending on the type of host. Preferable examples of the expression vector include those that are capable of self-replicating in host cells or of being introduced into the chromosome of the host, and that contain a promoter in a position to which a nucleic acid encoding a target protein can be transferred.

As the host, any of prokaryotes, and eukaryotes such as yeast, filamentous fungi, insect cells, animal cells, and plant cells, can be suitably used.

Preferable examples of prokaryotic hosts include bacteria belonging to the genera Escherichia, Brevibacillus, Serratia, Bacillus, Microbacterium, Brevibacterium, Corynebacterium, Pseudomonas, and the like. Examples of microorganisms belonging to the genus Escherichia include Escherichia coli, etc. Examples of microorganisms belonging to the genus Brevibacillus include Brevibacillus agri, etc. Examples of microorganisms belonging to the genus Serratia include Serratia liquefaciens, etc. Examples of microorganisms belonging to the genus Bacillus include Bacillus subtilis, etc. Examples of microorganisms belonging to the genus Microbacterium include Microbacterium ammoniaphilum, etc. Examples of microorganisms belonging to the genus Brevibacterium include Brevibacterium divaricatum, etc. Examples of microorganisms belonging to the genus Corynebacterium include Corynebacterium ammoniagenes, etc. Examples of microorganisms belonging to the genus Pseudomonas include Pseudomonas putida, etc.

When a prokaryotic host is used, examples of the vector for introducing a nucleic acid encoding a target protein include pBTrp2 (commercially available from Boehringer Mannheim), pGEX (commercially available from Pharmacia), pUC18, pBluescriptII, pSupex, pET22b, pCold, pUB110, and pNCO2 (Japanese Unexamined Patent Publication No. 2002-238569), and the like.

Examples of eukaryotic hosts include yeast and filamentous fungi (mold etc.). Examples of yeast include yeast belonging to the genera Saccharomyces, Pichia, Schizosaccharomyces, and the like. Examples of filamentous fungi include filamentous fungi belonging to the genera Aspergillus, Penicillium, Trichoderma, and the like.

When a eukaryotic host is used, examples of the vector for introducing a nucleic acid encoding a target protein include YEP13 (ATCC37115), YEp24 (ATCC37051), and the like. The method for introducing an expression vector into the abovementioned host cells may be any method as long as it is a method for introducing DNA into the host cells. Examples of the method include a method using calcium ions (Proc. Natl. Acad. Sci. USA, 69, 2110 (1972)), an electroporation method, a spheroplast method, a protoplast method, a lithium acetate method, a competent method, and the like.

The method for expressing the nucleic acid by a host transformed with an expression vector may be direct expression. In addition, secretory production, fusion protein expression, etc., can be performed according to the method described in the 2nd Edition of Molecular Cloning (Cold Spring Harbor Laboratory, 1989).

The protein can be produced by, for example, culturing a host transformed with an expression vector in a culture medium, allowing the production and accumulation of the protein in the culture medium, and harvesting the protein from the culture medium. The method for culturing the host in the culture medium can be performed according to a process generally used for host culture.

When the host is a eukaryote such as Escherichia coli or a prokaryote such as yeast, the culture medium may be a natural medium or a synthetic medium as long as it contains a carbon source, a nitrogen source, an inorganic salt, etc. that can be assimilated by the host and the host can be efficiently cultured.

The carbon source may be one that can be assimilated by the abovementioned transformed host. Examples thereof include glucose, fructose, sucrose, and molasses containing them; carbohydrates such as starch and starch hydrolysates; organic acids such as acetic acid and propionic acid; and alcohols such as ethanol and propanol. Examples of the nitrogen source include ammonia, ammonium salts of inorganic acids or organic acids such as ammonium chloride, ammonium sulfate, ammonium acetate, and ammonium phosphate; other nitrogen-containing compounds; peptone, meat extract, yeast extract, corn steep liquor, casein hydrolysate, soybean cake, soybean cake hydrolyzate, various fermentative bacteria and digests thereof. Usable examples of inorganic salts include monopotassium phosphate, dipotassium phosphate, magnesium phosphate, magnesium sulfate, sodium chloride, ferrous sulfate, manganese sulfate, copper sulfate, and calcium carbonate.

Prokaryotes such as Escherichia coli or eukaryotes such as yeast can be cultured under aerobic conditions by shaking culture or aeration agitation submerged culture, for example. The culture temperature is 15 to 40°C, for example. The culture time is generally 16 hours to 7 days. The pH of the culture medium during culture is preferably maintained at 3.0 to 9.0. The pH of the culture medium can be adjusted using inorganic acids, organic acids, alkali solutions, urea, calcium carbonate, ammonia, etc.

Moreover, antibiotics, such as ampicillin and tetracycline, may be added to the culture medium during culture, if necessary. When a host transformed with an expression vector using an inducible promoter as a promoter is cultured, an inducer may be added to the medium, if necessary. For example, when a host transformed with an expression vector using a lac promoter is cultured, isopropyl-p-D-thiogalactopyranoside or the like may be added to the medium; and when a host transformed with an expression vector using a trp promoter is cultured, indole acrylate or the like may be added to the medium.

(Isolation and purification of proteins)

The expressed protein can be isolated and purified by a generally used method. For example, when the protein is expressed in a soluble state in the cells, the host cells are collected by centrifugal separation after completion of the culture, and suspended in a water-based buffer. Then, the host cells are disrupted by an ultrasonic disruption machine, a French press, a Manton-Gaulin homogenizer, a Dyno-Mill, etc., and a cell-free extract is obtained. The cell-free extract is centrifuged to obtain a supernatant, from which a purified preparation can be obtained by methods generally used for the isolation and purification of proteins, all of which can be used singly or in combination, such as a solvent extraction method, a salting-out method using ammonium sulfate etc., a desalination method, a precipitation method using an organic solvent, an anion-exchange chromatography method using resins such as diethylaminoethyl (DEAE)-sepharose and DIAION HPA-75 (commercially available from Mitsubishi Chemical Corp.), a cation-exchange chromatography method using resins such as S-Sepharose FF (commercially available from Pharmacia), a hydrophobic chromatography method using resins such as butyl sepharose and phenyl sepharose, a gel-filtration method using molecular sieving, an affinity chromatography method, a chromatofocusing method, and an electrophoresis method such as isoelectric focusing.

Moreover, when the protein is expressed while forming insoluble fractions in the cells, the insoluble fractions of the protein are collected as precipitation fractions by similarly collecting the host cells, followed by disruption and centrifugal separation. The collected insoluble fractions of the protein can be solubilized by a protein modifier. After this operation, a purified preparation of the protein can be obtained by the same isolation and purification method as described above. When the protein is secreted outside the cells, the protein can be collected from the culture supernatant. More specifically, the culture is treated by centrifugal separation or like method to obtain a culture supernatant, and a purified preparation can be obtained from the culture supernatant by the same isolation and purification method as described above.

(Protein molded article)

A protein molded article according to the present embodiment may be, for example, an article obtained by molding the above proteins into an arbitrary shape. The protein molded article according to the present embodiment may contain inevitable components, for example, impurities contained in the proteins. The shape of the protein molded article is not particularly limited, and examples thereof include fibers, films, gels, and porous bodies. The protein molded article according to the present embodiment includes an ionic liquid therein.

(Ionic liquid)

The ionic liquid contained in the protein molded article according to the present embodiment is not particularly limited, but it may be, for example, an organic salt that becomes a liquid at 100°C or lower. Examples of ionic liquids include salts with cations such as imidazolium, pyrrolidinium, piperidinium, pyridinium, quaternary ammonium, quaternary phosphonium, sulfonium, and their derivatives, and anions such as halogens, triflate, tetrafluoroborate, hexafluorophosphate, and carboxylic acid. In consideration of solubility of proteins, an imidazolium salt including imidazolium or its derivative as a cation is preferably used.

Examples of imidazolium salts include 1,3-dimethylimidazolium salts, 1-ethyl-3-methylimidazolium salts, 1-methyl-3-propylimidazolium salts, 1-butyl-3-methylimidazolium salts, 1-hexyl-3-methylimidazolium salts, 1-methyl-3-n-octylimidazolium salts, 1-(2-hydroxyethyl)-3-methylimidazolium salts, 1-decyl-3-methylimidazolium salts, 1-ethyl-2,3-dimethylimidazolium salts, 1,2-dimethyl-3-propylimidazolium salts, 2,3-dimethyl-1-propylimidazolium salts, 1-butyl-2,3-dimethylimidazolium salts, and 1-hexyl-2,3-dimethylimidazolium salts, and 1-ethyl-3-methylimidazolium salts, 1-butyl-3-methylimidazolium salts, and 1-hexyl-3-methylimidazolium salts are preferable.

The ionic liquid may be a hydrophobic ionic liquid or a hydrophilic ionic liquid. Examples of hydrophobic ionic liquids include 1-ethyl-3-methylimidazolium bis(trifluoromethanesulfonyl)imide ([Emim][TFSI]), 1-butyl-3-methylimidazolium bis(trifluoromethanesulfonyl)imide ([Bmim][TFSI]), 1-hexyl-3-methylimidazolium bis(trifluoromethanesulfonyl)imide ([Hmim][TFSI]), 1-decyl-3-methylimidazolium bis(trifluoromethanesulfonyl)imide ([Dmim][TFSI]), 1-hexyl-3-methylimidazolium trifluoromethanesulfonate ([Hmim][OTf]), 1-methyl-3-n-octylimidazolium trifluoromethanesulfonate ([Omim][OTf]), and 1-hexyl-3-methylimidazolium chloride ([Hmim][Cl]). When a hydrophobic ionic liquid is used, it is possible to further curb separation of the ionic liquid from the protein molded article. Examples of hydrophilic ionic liquids include 1-ethyl-3-methylimidazolium chloride ([Emim][Cl]), 1-butyl-3-methylimidazolium chloride ([Bmim][Cl]), 1-ethyl-3-methylimidazolium dimethyl phosphate ([Emim][DMP]),1-ethyl-3-methylimidazolium diethyl phosphate ([Emim][DEP]), 1-ethyl-3-methylimidazolium acetate ([Emim][Acet]), 1-butyl-3-methylimidazolium acetate ([Bmim][Acet]), and 1-butyl-3-methylimidazolium hexafluorophosphate ([Bmim][PF6]). When a hydrophilic ionic liquid is used, it is possible to more easily prepare a solution for molding a protein molded article and a protein solution.

The ionic liquid generally has a very low vapor pressure and is non-volatile. Therefore, the protein molded article according to the present embodiment can stably hold the ionic liquid therein and can stably maintain an effect of improving flexibility and elongation.

As the ionic liquid, an ionic liquid, having a temperature at which a heat weight loss ratio is 10% by mass is 200°C or higher, may be used. Therefore, the protein molded article according to the present embodiment can stably maintain an effect of improving flexibility and elongation under a high temperature environment. The heat weight loss ratio is a value calculated from the change in weight from when heating is started when the ionic liquid is heated at a quasi-static speed (5°C/min) from 50 to 500°C.

(Content of ionic liquid in protein molded article)

The content of the ionic liquid in the protein molded article according to the present invention is not particularly limited and may be appropriately set according to the type of the protein molded article, the type of the ionic liquid, the type of the protein, and the like. The content of the ionic liquid in the protein molded article exceeds, for example, 0% by mass, and is in a range in which the protein molded article can be molded (for example, 65% by mass).

[Method for producing protein molded article]

The protein molded article according to the present embodiment can be produced by, for example, a method in which an ionic liquid is mixed into a molding material of a protein molded article and molding is then performed, a method in which an ionic liquid is immersed in a molding material of a protein molded article and molding is then performed, a method in which a molded article molded according to a general method is immersed in an ionic liquid, or the like. Examples of a method in which an ionic liquid is mixed into a molding material of a protein molded article and molding is then performed include a method in which a solution obtained by adding an ionic liquid to a solution in which proteins are dissolved (molding solution) or a solution obtained by additionally adding a solvent for dissolving proteins into a mixture in which protein powder and an ionic liquid are mixed is used as a molding material (molding solution), molding is performed according to a general method, and thus a protein molded article is obtained. In addition, examples of a method in which an ionic liquid is immersed in a molding material of a protein molded article and molding is then performed include a method in which a material obtained by adding an ionic liquid to protein powder (molding material) and mixing them, and introducing an ionic liquid into powder based on liquid absorption of the protein powder is used as a molding material, molding is performed according to a predetermined method, and thus a protein molded article is obtained.

(Molding material of protein molded article)

The molding material of a protein molded article may be a molding solid material containing protein powder or a molding solution in which proteins are dissolved in a solvent. The molding solution includes proteins and a solvent.

(Solvent)

The solvent used for a molding solution is not particularly limited as long as it can dissolve proteins. Examples thereof include protic polar solvents such as hexafluoro-2-propanol (HFIP) and formic acid, and non-protic polar solvents such as dimethyl sulfoxide (DMSO), N,N-dimethylformamide (DMF), and N,N-dimethylacetamide (DMAc).

The molding solution may further include inorganic salts as a dissolution promoter. Examples of inorganic salts include inorganic salts composed of Lewis acids and Lewis bases. Examples of Lewis bases include oxo acid ions (nitrate ions, perchlorate ions, etc.), metal oxo acid ions (permanganate ions etc.), halide ions, thiocyanate ions, cyanate ions, and the like. Examples of Lewis acids include metal ions such as alkali metal ions and alkaline earth metal ions; polyatomic ions such as ammonium ions; complex ions; and the like. Specific examples of inorganic salts composed of Lewis acids and Lewis bases include lithium salts such as lithium chloride, lithium bromide, lithium iodide, lithium nitrate, lithium perchlorate, and lithium thiocyanate; calcium salts such as calcium chloride, calcium bromide, calcium iodide, calcium nitrate, calcium perchlorate, and calcium thiocyanate; iron salts such as iron chloride, iron bromide, iron iodide, iron nitrate, iron perchlorate, and iron thiocyanate; aluminum salts such as aluminum chloride, aluminum bromide, aluminum iodide, aluminum nitrate, aluminum perchlorate, and aluminum thiocyanate; potassium salts such as potassium chloride, potassium bromide, potassium iodide, potassium nitrate, potassium perchlorate, and potassium thiocyanate; sodium salts such as sodium chloride, sodium bromide, sodium iodide, sodium nitrate, sodium perchlorate, and sodium thiocyanate; zinc salts such as zinc chloride, zinc bromide, zinc iodide, zinc nitrate, zinc perchlorate, and zinc thiocyanate; magnesium salts such as magnesium chloride, magnesium bromide, magnesium iodide, magnesium nitrate, magnesium perchlorate, and magnesium thiocyanate; barium salts such as barium chloride, barium bromide, barium iodide, barium nitrate, barium perchlorate, and barium thiocyanate; strontium salts such as strontium chloride, strontium bromide, strontium iodide, strontium nitrate, strontium perchlorate, and strontium thiocyanate; and the like.

The viscosity of the molding solution may be appropriately set. When the molding solution is used as a dope solution, the viscosity of the molding solution can be, for example, 100 to 15,000 cP (centipoises) at 35°C. The viscosity of the molding solution can be measured using, for example, "EMS viscometer" (product name, commercially available from Kyoto Electronics Manufacturing Co., Ltd.).

When the ionic liquid is mixed into the molding solution in advance, in order to further improve an amount of proteins dissolved, the content of the ionic liquid is preferably 10 to 20% by mass based on a total amount of the molding solution.

The content of proteins in the molding solution can be 15% by mass or more, 30% by mass or more, 40% by mass or more, or 50% by mass or more based on a total amount of the molding solution. In consideration of production efficiency of the molding solution, the content of proteins can be 70% by mass or less, 65% by mass or less, or 60% by mass or less based on a total amount of the molding solution.

Regarding specific examples of a method for producing a protein molded article according to the present embodiment, cases in which a protein molded article is a protein fiber, a protein film, a protein gel, a protein porous body and a protein molded article will be exemplified below.

(Method for producing protein fibers)

Protein fibers according to the present embodiment are obtained by spinning the above proteins, and include the above proteins as a main component. Protein fibers can be produced using the above molding solution as a dope solution according to a known spinning method. That is, for example, when protein fibers including spider silk fibroins as a main component are produced, first, spider silk fibroins produced according to the above method for producing proteins are added to a solvent such as dimethyl sulfoxide (DMSO), N,N-dimethylformamide (DMF), formic acid or hexafluoro-2-propanol (HFIP) together with an inorganic salt as a dissolution promoter and dissolved to produce a dope solution. The ionic liquid may be added to the dope solution in advance. Next, using this dope solution, spinning is performed according to a known spinning method such as wet spinning, dry spinning, or dry and wet spinning, and thus desired protein fibers can be obtained. When the dope solution includes the ionic liquid, desired protein fibers can also be obtained in the same manner.

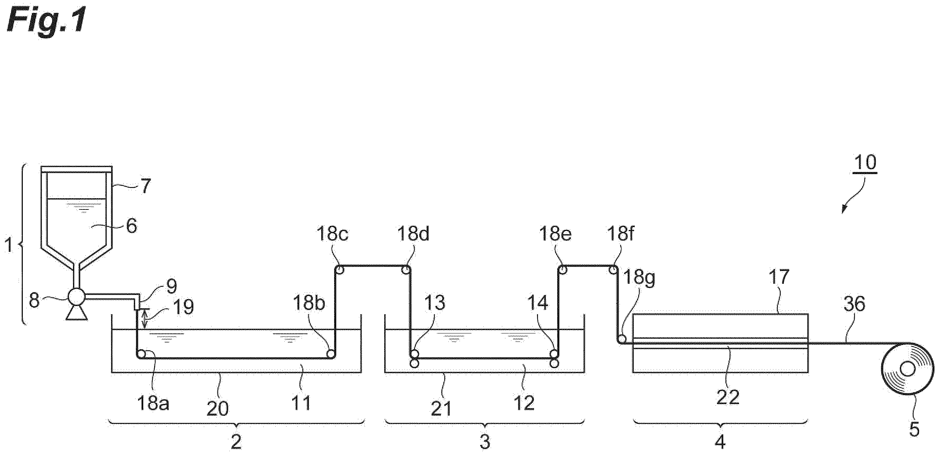

FIG. 1 is a schematic view showing an example of a spinning device for producing protein fibers. The spinning device 10 shown in FIG. 1 is an example of a spinning device for dry and wet spinning, and includes an extrusion device 1, a coagulation bathtub 20, a washing bathtub 21, and a drying device 4 in order from the upstream end.

The extrusion device 1 includes a storage tank 7 in which a dope solution (spinning dope) 6 is stored. A coagulating liquid 11 (for example, methanol, saturated citric acid (water/ethanol = 1:1, mass/mass)) is stored in the coagulation bathtub 20. The dope solution 6 is discharged from a nozzle 9 with an air gap 19 between it and the coagulating liquid 11 by a gear pump 8 attached to a lower end of the storage tank 7. The discharged dope solution 6 is supplied into the coagulating liquid 11 via the air gap 19. The solvent is removed from the dope solution 6 in the coagulating liquid 11 to coagulate proteins. The coagulated proteins are guided to the washing bathtub 21, washed with a washing solution 12 in the washing bathtub 21, and then sent to the drying device 4 by a first nip roller 13 and a second nip roller 14 installed in the washing bathtub 21. In this case, for example, when a rotational speed of the second nip roller 14 is set to be higher than a rotational speed of the first nip roller 13, protein fibers 36 stretched at a ratio corresponding to a rotational speed ratio are obtained. The protein fibers stretched in the washing solution 12 are released from the washing bathtub 21, then dried while passing through the drying device 4, and then wound around a winder. Thus, a wound product 5 in which protein fibers are finally wound around a winder by the spinning device 10 is obtained. Here, 18a to 18g indicate yarn guides. In addition, when the dope solution includes the ionic liquid, the obtained protein fibers contain the ionic liquid dispersed therein.

The coagulating liquid 11 may be any solution that can be desolvated, and examples thereof include lower alcohols having 1 to 5 carbon atoms such as methanol, ethanol, and 2-propanol, glycol solvents such as ethylene glycol, propylene glycol, and glycerol, and acetone. The coagulating liquid 11 may appropriately include water.

The coagulating liquid 11 may further include an ion supply substance that supplies ions into the coagulating liquid. Examples of ion supply substances include inorganic salts such as calcium chloride, ammonium sulfate, and magnesium chloride, and organic acids such as citric acid and oxalic acid. When the coagulating liquid includes an inorganic salt or an organic acid, due to its osmotic pressure, separation of the ionic liquid from protein fibers in the coagulation bath can be curbed. When the coagulating liquid includes an organic acid, separation of the ionic liquid can be curbed and also aggregation of proteins can be promoted. In this regard, it is preferable that the ion supply substance such as an inorganic salt and an organic acid be contained in a saturated amount in the coagulating liquid.

The coagulating liquid 11 is preferably an ethanol solution including a saturated amount of an inorganic salt or organic acid (a solution including 50% by mass or more of ethanol) or an isopropanol solution including a saturated amount of an organic acid, and more preferably a water-ethanol solution including a saturated amount of citric acid (saturated citric acid solution (water/ethanol = 1:1, mass/mass)) or an isopropanol solution including a saturated amount of citric acid.

The temperature of the coagulating liquid 11 is preferably 0 to 30°C. A distance that the coagulated protein passes through the coagulating liquid 11 (substantially, a distance from the yarn guide 18a to the yarn guide 18b) may be any length at which desolvation is efficiently performed, and is, for example, 200 to 500 mm. A time for which it is retained in the coagulating liquid 11 may be, for example, 0.01 to 3 minutes, and preferably 0.05 to 0.15 minutes. In addition, stretching (pre-stretching) may be performed in the coagulating liquid 11.

Protein fibers can be made into a stretched yarn after a stretching step. Examples of a stretching method include wet heat stretching and dry heat stretching.

Wet heat stretching may be performed in the washing bathtub 21 when protein fibers are obtained. Wet heat stretching is performed in hot water, a solution in which an organic solvent and the like are added to hot water, or the like. The wet heat stretching temperature may be, for example, 50 to 90°C, and is preferably 75 to 85°C. In the wet heat stretching, an unstretched yarn can be stretched, for example, to 1 to 10 times its length, and is preferably stretched to 2 to 8 times its length.

Dry heat stretching can be performed using an electric tubular furnace, a dry heat plate, or the like. The temperature may be, for example, 140°C to 270°C, and is preferably 160°C to 230°C. In the dry heat stretching, an unstretched yarn can be stretched, for example, to 0.5 to 8 times its length, and is preferably stretched to 1 to 4 times its length.

Wet heat stretching and dry heat stretching may be performed independently or may be performed in multiple stages, or in combination. That is, wet heat stretching and dry heat stretching may be appropriately combined, for example, wet heat stretching is performed in the first stage stretching and dry heat stretching is performed in the second stage stretching, or wet heat stretching is performed in the first stage stretching and wet heat stretching is performed in the second stage stretching, and additionally, dry heat stretching is performed in the third stage stretching.

The lower limit value of the final stretch ratio is preferably any of more than 1, 2 or more, 3 or more, 4 or more, 5 or more, 6 or more, 7 or more, 8 or more, and 9 or more with respect to the unstretched yarn. The upper limit value is preferably 40 or less, 30 or less, 20 or less, 15 or less, 14 or less, 13 or less, 12 or less, 11 or less, or 10 or less.

When the dope solution does not include the ionic liquid, the molded article molded according to, for example, the above method, is immersed in the ionic liquid, and thus protein fibers including the ionic liquid dispersed therein can be obtained.

(Method for producing protein film)

The protein film according to the present embodiment can be produced using the above molding solution as a dope solution and by cast-molding the dope solution on the surface of the substrate, and performing drying and/or desolvation. The ionic liquid may be added to the dope solution in advance. When the dope solution includes the ionic liquid, a desired protein film can also be obtained in the same manner.

Specifically, first, the dope solution is applied to the surface of the substrate at a predetermined thickness (for example, the thickness after drying and/or desolvation is 1 to 1000 μm).

The substrate may be a resin substrate, a glass substrate, a metal substrate, or the like. The substrate is preferably a resin substrate so that a film after cast molding can be easily separated. The resin substrate may be, for example, a polyethylene terephthalate (PET) film, a fluorine resin film of polytetrafluoroethylene or the like, a polypropylene (PP) film, or a release film in which a silicone compound is fixed to the surface of such a film. The substrate is more preferably a PET film or a release film in which a silicone compound is fixed to the surface of a PET film because it is stable with respect to a DMSO solvent, a dope solution can be stably cast-molded, and a film after molding can be easily separated.

It is preferred that the drying and/or desolvation are performed, for example, by at least one means selected from vacuum drying, hot-air drying, air drying, and immersion. The immersion for desolvation of cast film may be performed in the coagulating liquid (desolvation liquid) described in the description of the method for producing the protein fiber. The temperature of the coagulating liquid (desolvation liquid) may be 0 to 200°C, preferably 0 to 90°C. Preferably, the solvent is removed as much as possible. In the case of stretching the film in liquid, desolvation can be performed simultaneously with stretching. Note that the desolvation may be performed after stretching the film.

The unstretched film after the drying and/or desolvation can be stretched uniaxially or biaxially in water. The biaxial stretching may be either sequential stretching or simultaneous biaxial stretching. Multistage stretching composed of two or more stages may be performed. The stretch ratio is preferably 1.01 to 6 times, and further preferably 1.05 to 4 times in both of the horizontal and vertical directions. Within this range, a balance between the stress and strain can be adjusted easily. The stretching in water is preferably at a water temperature of 20 to 90°C. The film after the stretching is preferably subjected to thermal fixation by dry heat at 50 to 200°C for 5 to 600 seconds. The thermal fixation provides the film with dimensional stability at ambient temperature. Incidentally, the film stretched uniaxially will be a uniaxially-oriented film, and the film stretched biaxially will be a biaxially-oriented film.

The film may be a color film. In this case, a colorant such as a dye is dissolved or dispersed, for example, in a DMSO solvent, to produce a DMSO coloring liquid, the coloring liquid and a dope solution are mixed to obtain a solution, and the solution is cast-molded in the same manner as above to produce a film. Then, the film is dried and/or desolvated to form an unstretched color film or stretched to form a stretched film. The color film can be applied to a reflective plate, a marker, a UV protection film, a slit yarn, and the like.

When the dope solution includes the ionic liquid, the obtained protein film includes the ionic liquid dispersed therein. When the dope solution does not include the ionic liquid, for example, the molded article molded according to the above method is immersed in the ionic liquid, and thus a protein film including the ionic liquid dispersed therein can be obtained.

(Method for producing protein gel)

The protein gel according to the present embodiment can be produced according to a production method using the above molding solution, which includes a step in which the solvent for the molding solution is substituted with a water-soluble solvent (substitution step) and a step in which a gel is molded into a predetermined shape as necessary (molding step). The ionic liquid may be added to the molding solution in advance. When the molding solution includes the ionic liquid, a desired protein gel can also be obtained in the same manner.

In the substitution step, the solvent in the molding solution is substituted with a water-soluble solvent. The water-soluble solvent is a solvent containing water, and examples thereof include water, a water-soluble buffer solution, and saline. The water-soluble solvent is preferably water because it has high adaptability to the human body. Water is not particularly limited, and pure water, distilled water, ultrapure water, or the like can be used.

The substitution step is preferably performed by a method in which a molding solution is put into a dialysis film and immersed in a water-soluble solvent, and the water-soluble solvent is replaced one or more times. Specifically, more preferably, a molding solution is put into a dialysis film and left in a water-soluble solvent (one batch) in an amount 100 times or more that of the molding solution for 3 hours, and replacement of the water-soluble solvent is repeated three or more times in total. The dialysis film may be any film that does not allow proteins to pass through, and may be, for example, a cellulose dialysis film. When substitution of the water-soluble solvent is repeated, the amount of the solvent in the molding solution can be brought close to zero. In the latter half of the step of substitution with a water-soluble solvent, the dialysis film may not be used.

The molding step is performed between the molding solution preparation step and the substitution step, and may be a step in which a molding solution is poured into a mold and molded into a predetermined shape or a step which is performed after the substitution step and in which a gel obtained in the substitution step is cut and molded into a predetermined shape.

As described above, for example, a protein gel having a moisture content of 85.0 to 99.9% by mass can be obtained. When the molding solution includes the ionic liquid, the obtained protein gel includes the ionic liquid dispersed therein. On the other hand, when the molding solution does not include the ionic liquid, for example, the molded article molded according to the above method is immersed in the ionic liquid, and thus a protein gel including the ionic liquid dispersed therein can be obtained.

(Method for producing protein porous body)

The protein porous body according to the present embodiment can be produced according to a production method using the above molding solution, which includes a step in which the solvent for the molding solution is substituted with a water-soluble solvent (substitution step) and a step in which a gel is molded into a predetermined shape as necessary (molding step). The ionic liquid may be added to the molding solution in advance. When the molding solution includes the ionic liquid, a desired protein porous body can also be obtained in the same manner.

Regarding the substitution step and the molding step, the same ones described in the method for producing a protein gel can be exemplified.

In the drying step, preferably, vacuum freeze-drying is adopted. The degree of vacuum at vacuum freeze-drying is preferably 200 Pa or less, more preferably 150 Pa or less, and further preferably 100 Pa or less. By vacuum drying, water evaporates from the gel, and the temperature declines by the evaporation latent heat, whereby it is brought into a frozen state. The temperature of the gel at vacuum freeze-drying is preferably 70°C or less, more preferably 60°C or less, and further preferably 50°C or less. Incidentally, prior to vacuum freeze-drying, the gel may be pre-frozen at a temperature of -10 to - 45°C for about 10 to 36 hours. The moisture content after freeze-drying is preferably 5.0% or less, and more preferably 3.0% or less.

As described above, for example, a protein porous body can be obtained. When the molding solution includes the ionic liquid, the obtained protein porous body includes the ionic liquid dispersed therein. On the other hand, when the molding solution does not include the ionic liquid, for example, the molded article molded according to the above method is immersed in the ionic liquid, and thus a protein porous body including the ionic liquid dispersed therein can be obtained.

(Method for producing protein molded article)

The protein molded article according to the present embodiment can be produced according to a general method using the above molding material (molding solid material). Specifically, first, the molding material is introduced into a mold of a pressure molding machine, the mold is then heated, and the molding material is pressurized. Heating and pressurization continue until the mixture reaches a predetermined temperature under a predetermined pressure and thereby a mixture in which the molding material is heated and pressurized is obtained. Next, the temperature of the mold is lowered using a cooler (for example, a spot cooler), and when the mixture reaches a predetermined temperature, the content can be removed to obtain a molded article. As the molding material (molding solid material), a material in which an ionic liquid is introduced in advance may be used. That is, a material in which an ionic liquid is added to protein powder and mixed, and enters the inside of the powder may be used.

The temperature when the mold is heated is preferably 80 to 300°C, more preferably 100 to 180°C, and most preferably 100 to 130°C. The pressure when the mold is pressurized is preferably 5 kN or more, more preferably 10 kN or more, and most preferably 20 kN or more. In addition, after predetermined heating and pressurizing conditions are satisfied, a time for which the treatment continues under the conditions (heat retention time) is preferably 0 to 100 minutes, more preferably 10 to 50 minutes, and most preferably 5 to 30 minutes.

As described above, for example, a protein molded article can be obtained. When the molding material includes the ionic liquid, the obtained protein molded article includes the ionic liquid dispersed therein. On the other hand, when the molding material does not include the ionic liquid, for example, the molded article molded according to the above method is immersed in the ionic liquid, and thus a protein molded article including the ionic liquid dispersed therein can be obtained.

[Protein solution]

The protein solution according to the present invention includes proteins, an ionic liquid, and a solvent. As the protein solution, the same one described regarding the molding solution including an ionic liquid can be exemplified. The protein solution according to the present embodiment can be prepared by adding an ionic liquid to a solution in which proteins are dissolved in a solvent (for example, a molding solution) and mixing them or can be prepared by adding an ionic liquid to protein powder and performing dissolving, and then additionally adding a solvent and mixing them. The protein solution according to the present embodiment can be preferably used for producing the protein molded article according to the present invention.

[Protein molded article plasticizer]

The protein molded article according to the present invention includes an ionic liquid therein, and thus the flexibility and elongation are improved. That is, the ionic liquid functions as a plasticizer for the protein molded article. Therefore, in one embodiment of the present invention, a protein molded article plasticizer including an ionic liquid is provided. The protein molded article plasticizer according to the present embodiment can impart excellent flexibility and elongation to the protein molded article. A preferable form of the ionic liquid, a preferable use method of a plasticizer (ionic liquid), and the like are as described above.

Examples

The present invention is described in more detail below based on Examples. However, the present invention is not limited to the following Examples.

[Test Example 1: Production and Evaluation of Protein Fiber]

<(1-1) Production of Spider Silk Protein (Spider Silk Fibroin: PRT799>

(Synthesis of Gene Encoding Spider Silk Protein, and Construction of Expression Vector)

Modified fibroin having the amino acid sequence represented by SEQ ID NO: 8 (hereinafter also referred to as "PRT799") was designed based on the base sequence and amino acid sequence of fibroin derived from Nephila clavipes (GenBank Accession Number: P46804.1, GI: 1174415).

The amino acid sequence represented by SEQ ID NO: 8 has an amino acid sequence with substitution, insertion, and deletion of amino acid residues in the amino acid sequence of fibroin derived from Nephila clavipes for the purpose of improving productivity, and the amino acid sequence represented by SEQ ID NO: 7 (tag sequence and hinge sequence) is further added to the N-terminal.

Next, a nucleic acid encoding PRT799 was synthesized. An NdeI site was added to the 5'-end of the nucleic acid, and an EcoRI site was added to the downstream of the stop codon. The nucleic acid was cloned into a cloning vector (pUC118). Thereafter, the nucleic acid was digested with restriction enzymes NdeI and EcoRI, and then recombined into a protein expression vector pET-22b(+). Thus, the expression vector was obtained.

Escherichia coli BLR(DE3) was transformed with the pET22b(+) expression vector containing the nucleic acid encoding PRT799. The transformed Escherichia coli was cultured in 2 mL of LB medium containing ampicillin for 15 hours. The culture solution was added to 100 mL of seed culture medium containing ampicillin (Table 1) so that the $OD_{600}$ was 0.005. The culture solution temperature was maintained at 30°C, and flask culture was performed (for about 15 hours) until the $OD_{600}$ reached 5, thereby obtaining a seed culture solution.

[Table 1]

| Seed culture medium | |
| --- | --- |
| Reagent | Concentration (g/L) |
| Glucose | 5.0 |
| $KH_2PO_4$ | 4.0 |
| $K_2HPO_4$ | 9.3 |
| Yeast Extract | 6.0 |
| Ampicillin | 0.1 |

The seed culture solution was added to a jar fermenter, to which 500 ml of production medium (Table 2 below) was added, so that the $OD_{600}$ was 0.05. The culture solution temperature was maintained at 37°C, and culture was performed while constantly controlling the pH at 6.9. Moreover, the dissolved oxygen concentration of the culture solution was maintained at 20% of the saturated dissolved oxygen concentration.

[Table 2]

| Production medium | |
| --- | --- |
| Reagent | Concentration (g/L) |
| Glucose | 12.0 |
| $KH_2PO_4$ | 9.0 |
| $MgSO_4 \cdot 7H_2O$ | 2.4 |
| Yeast Extract | 15 |
| $FeSO_4 \cdot 7H_2O$ | 0.04 |
| $MnSO_4 \cdot 5H_2O$ | 0.04 |
| $CaCl_2 \cdot 2H_2O$ | 0.04 |
| ADEKANOL (Adeka, LG-295S) | 0.1(mL/L) |

Immediately after glucose in the production medium was completely consumed, a feed solution (glucose 455 g/l L, yeast extract 120 g/l L) was added at rate of 1 mL/min. The culture solution temperature was maintained at 37°C, and culture was performed while constantly controlling the pH at 6.9. Moreover, the dissolved oxygen concentration of the culture solution was maintained at 20% of the saturated dissolved oxygen concentration, and culture was performed for 20 hours. Thereafter, 1M isopropyl-β-thiogalactopyranoside (IPTG) was added to the culture solution to a final concentration of 1 mM, and the expression of PRT799 was induced. After the lapse of 20 hours since the adding of IPTG, the culture solution was centrifuged, and bacterial cells were collected. SDS-PAGE was carried out using bacterial cells prepared from the culture solution before and after IPTG was added, and the expression of PRT799 was confirmed by the appearance of a band of a size corresponding to PRT799 depending on IPTG addition.

(Purification of PRT799)

The bacterial cells which were collected 2 hours after the addition of IPTG were then washed with 20 mM Tris-HCl buffer (pH 7.4). The washed bacterial cells were suspended in 20 mM Tris-HCl buffer (pH 7.4) containing about 1 mM PMSF, and the cells were disrupted with a high-pressure homogenizer (commercially available from GEA Niro Soavi). The disrupted cells were centrifuged to obtain a precipitate. The obtained precipitate was washed with 20 mM Tris-HCl buffer (pH 7.4) to high purity. The precipitate after washing was suspended in 8M guanidine buffer (8M guanidinium hydrochloride, 10 mM sodium dihydrogen phosphate, 20 mM NaCl, 1 mM Tris-HCl, pH 7.0) to a concentration of 100 mg/mL, and was dissolved by stirring with a stirrer at 60°C. for 30 minutes. After dissolution, dialysis was carried out against water using a dialysis tube (Cellulose Tube 36/32, commercially available from Sanko Junyaku Co., Ltd.). White aggregated protein (PRT799) obtained after dialysis was collected by centrifugal separation, moisture was removed by a freeze dryer, and a freeze-dried powder was collected.

The degree of purification of PRT799 in the obtained freeze-dried powder was confirmed by image analysis of the results of polyacrylamide gel electrophoresis of the powder using Totallab (Nonlinear Dynamics Ltd.). As a result, the degree of purification of PRT799 was about 85%.

<(1-2) Production of protein fibers>

(Preparation of dope solution)

1-Ethyl-3-methylimidazolium dimethyl phosphate ([Emim][DMP]) was added as an ionic liquid to the spider silk fibroin (PRT799) obtained in (1-1) and N,N-dimethylacetamide (DMAc) was then additionally added thereto to produce a raw material liquid mixture. The concentration of the spider silk fibroin in the raw material liquid mixture was 22% by mass, and the concentration of the ionic liquid was 15% by mass. Then, the raw material liquid mixture was heated at 95°C for 1.5 hours with stirring, the spider silk fibroin was dissolved, and thereby a dope solution was obtained (the composition of the dope solution: PRT799/[Emim][DMP]/DMAc = 22/15/63 (mass ratio)).

(Spinning)

The dope solution obtained as above was subjected to dry and wet spinning using a spinning device corresponding to the spinning device 10 shown in FIG. 1 to obtain protein fibers. The dry and wet spinning was performed under the following conditions.

Under the conditions of an inner diameter of a nozzle (disposable needle) of a discharge part of 0.3 mm, a coagulating liquid (saturated citric acid solution (water/ethanol = 1 : 1, mass/mass)), a drying temperature of 60°C, and a stretch ratio of 2.4, protein fibers (Example 1) that had passed through a washing bathtub only once and protein fibers (Example 2) that had passed through a washing bathtub twice were produced.

<(1-3) Evaluation of ionic liquid content in protein fibers>

Cross sections of the protein fibers of Examples 1 and 2 were imaged and elemental analysis thereof was performed using a scanning electron microscope including an energy dispersive X-ray analyzing device (JSM-7100F, commercially available from JEOL Ltd.).

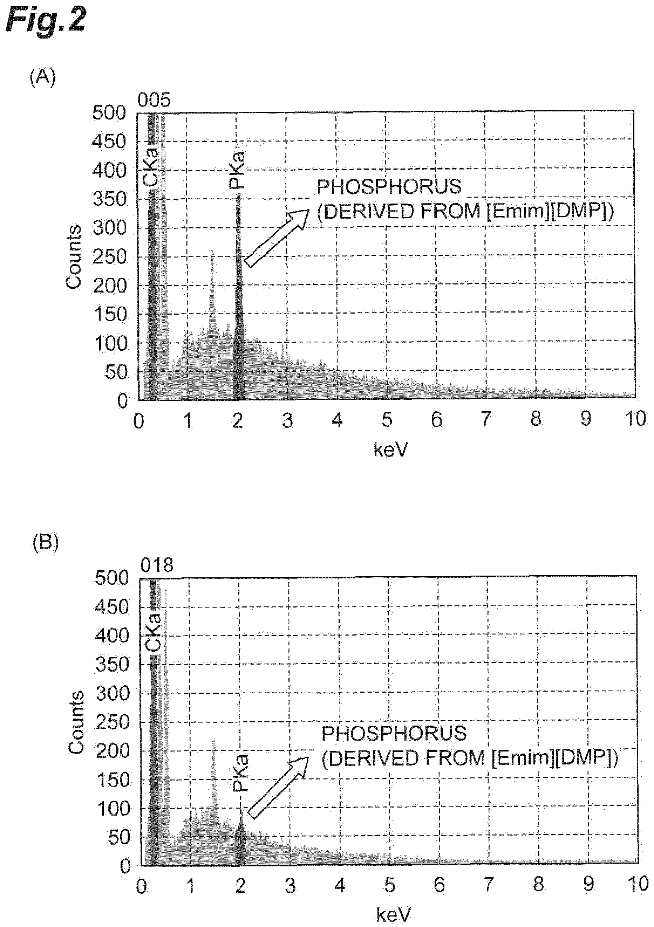

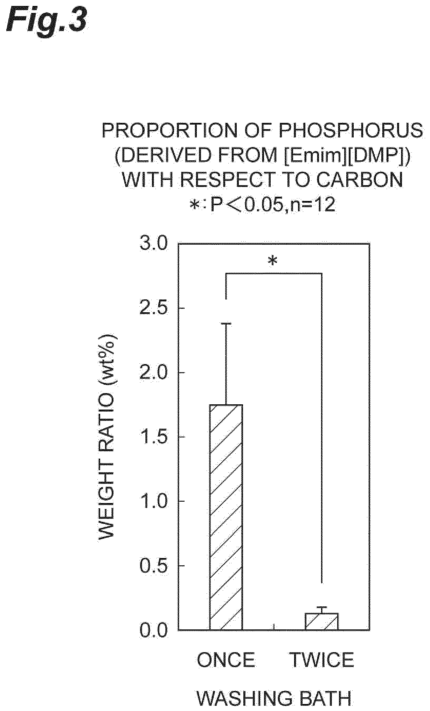

The results are shown in FIG. 2 and FIG. 3. FIG. 2(A) shows an energy dispersive X-ray analysis (EDX) spectrum of a cross section of protein fibers of Example 1, and FIG. 2(B) shows an EDX spectrum of a cross section of protein fibers of Example 2. In addition, FIG. 3 is a graph showing a proportion of phosphorus (derived from [Emim] [DMP]) with respect to carbon in the cross section of the protein fibers of Example 1 and Example 2.

As shown in FIGS. 2(A) and 2(B), in the EDX spectrum (point analysis) of the cross section of the protein fibers of Examples 1 and 2, a peak was observed at 2.0 keV derived from phosphorus of [Emim][DMP] used as the ionic liquid. In addition, as shown in FIG. 3, the protein fibers of Example 1 that were washed (passed through a washing bathtub) a small number of times after coagulation had a significantly higher proportion of phosphorus (derived from [Emim] [DMP]) with respect to carbon than the protein fibers of Example 2. Based on these results, it can be understood that the ionic liquid remaining (contained) in the protein fibers of Examples 1 and 2, and the protein fibers of Example 1 that were washed a small number of times after coagulation had a higher ionic liquid content.

<(1-4) Tensile test of protein fibers>

The protein fibers of Examples 1 and 2 were fixed with an adhesive to a piece of test paper having a distance between gripping tools of 20 mm, and under conditions of a temperature of 20°C and a relative humidity of 65%, stress and elongation were measured at a tensile speed of 10 cm/min using a tensile testing machine 3342 (commercially available from Instron). The load cell capacity was 10 N, and the gripping tool was of a clip type.

The toughness was obtained according to the following calculation formula.

$$\text{Toughness} = [E/(r^2 \times \pi \times L) \times 1000] \text{ (unit: MJ/m}^3\text{)}.$$

Here,

E represents a breaking energy (unit: J),
r represents a radius (unit: mm) of a fiber,
$\pi$ represents the ratio of the circumference of a circle to its diameter, and
L represents a distance between gripping tools during tensile test measurement (20 mm)

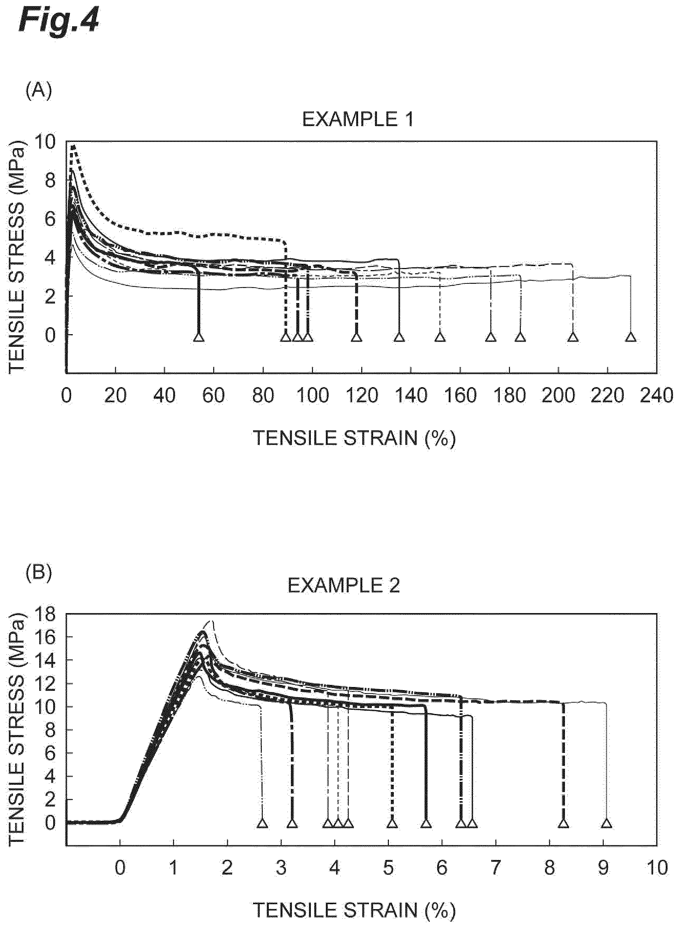

The results are shown in FIG. 4 and Table 3. FIG. 4 is a graph showing the results of the tensile test of the protein fibers of Example 1 and Example 2. In the graph of FIG. 4, the vertical axis represents the tensile stress (MPa), and the horizontal axis represents the tensile strain (%). Table 3 shows values of the tensile stress, tensile strain, Young's modulus, and toughness determined from the results of the tensile test (average value of the number of samples n = 11).

[Table 3]

| | Example 1 | | Example 2 | |
|---|---|---|---|---|
| | Average | S.D. | Average | S.D. |
| Diameter (long side width, $\mu$m) | 167.3 | 8.7 | 179.4 | 13.5 |
| Tensile stress (MPa) | 7.1 | 1.4 | 15.0 | 1.4 |
| Tensile strain (%) | 139 | 55 | 5 | 2 |
| Young's modulus (GPa) | 0.53 | 0.11 | 1.09 | 0.09 |
| Toughness (MJ/m$^3$) | 5.07 | 1.54 | 0.55 | 0.23 |

The protein fibers of Example 1 having a high ionic liquid content had a significantly higher value of the tensile strain than the protein fibers of Example 2. That is, it can be understood that the ionic liquid plasticized the protein fibers.

[Test Example 2: Production and Evaluation of Protein Film]

<(2-1) Production of Protein Film>

1-Ethyl-3-methylimidazolium dimethyl phosphate ([Emim][DMP]) was added as an ionic liquid to the spider silk fibroin (PRT799) obtained in (1-1) of Test Example 1 and N,N-dimethylacetamide (DMAc) was then additionally added thereto to produce a raw material liquid mixture. The concentration of the spider silk fibroin in the raw material liquid mixture was 22% by mass, and the concentration of the ionic liquid was 15% by mass. Then, the raw material liquid mixture was heated at 95°C for 1.5 hours with stirring, the spider silk fibroin was dissolved, and thereby a protein solution was obtained (the composition: PRT799/[Emim][DMP]/DMAc = 22/15/63 (mass ratio)).

0.5 mL of the protein solution was cast on a glass substrate so that the thickness was 200 $\mu$m, and the substrate

was heated at 160°C for 10 minutes, dried, and desolvated to obtain a protein film. The film thickness of the obtained protein film was 100 to 200 μm.

<(2-2) Evaluation of content of ionic liquid in protein film>

In the same manner as in (1-3) of Test Example 1, the cross section of the protein film was imaged and elemental analysis was performed.

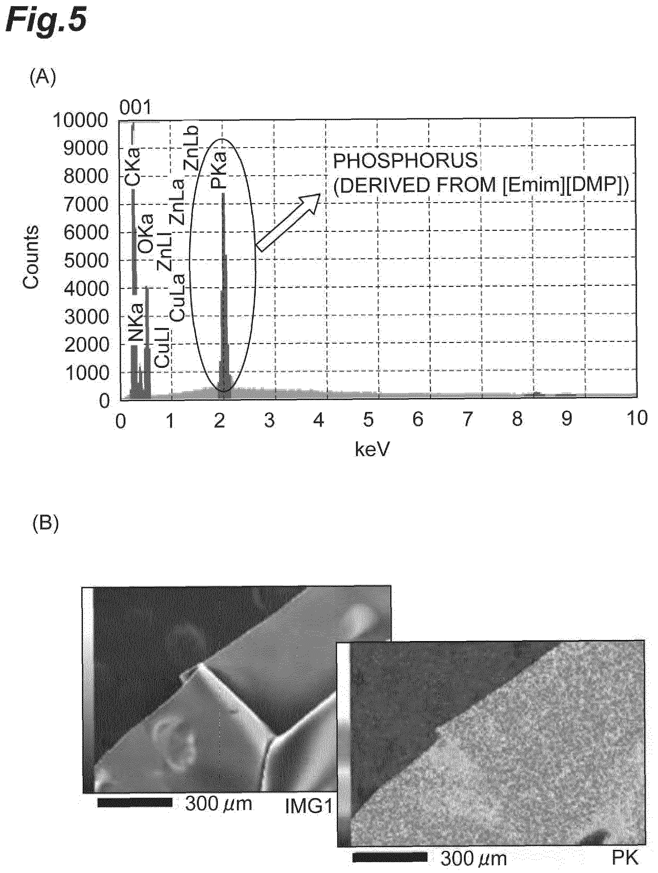

The results are shown in FIG. 5. FIG. 5(A) shows an EDX spectrum of a cross section of a protein film. FIG. 5(B) is a diagram showing results of surface analysis (phosphorus abundance) according to EDX of the cross section of the protein film.

As shown in FIG. 5(A), in the EDX spectrum (point analysis) of the cross section of the protein film, a peak was observed at 2.0 keV derived from phosphorus of [Emim][DMP] used as the ionic liquid. In addition, as shown in FIG. 5(B), phosphorus was distributed in the protein film. It can be understood that, since phosphorus was derived from the ionic liquid, the ionic liquid was dispersed and contained in the protein film.

[Test Example 3: Production and evaluation of protein solution]

The influence of the composition of the ionic liquid and the solvent on the solubility when the protein solution including the spider silk fibroin (PRT799) obtained in (1-1) of Test Example 1 was prepared was evaluated.

Specifically, preparation of solutions of Examples 3 and 4 and Comparative Examples 1 to 4 was attempted according to the compositions and composition ratios (weight ratios) shown in Table 4 and Table 5. As the ionic liquid, 1-butyl-3-methylimidazolium acetate ([Bmim][Acet]) and 1-ethyl-3-methylimidazolium dimethyl phosphate ([Emim][DMP]) were used. As the solvent, dimethylformamide (DMF) and dimethylacetamide (DMAc) were used. First, according to the composition ratios shown in Table 4 and Table 5, respective components were weighed in a glass container, and then heated at 100°C for 1 hour with stirring, and the solubility of the spider silk fibroin was visually checked.

[Table 4]

| | Weight ratio | | |
|---|---|---|---|
| | PRT799 | [Bmim][Acet] | DMF |
| BLANK | - | 1 | 3 |
| Comparative Example 1 | 1 | 1 | - |
| Comparative Example 2 | 1 | - | 3 |
| Example 3 | 1 | 1 | 3 |

[Table 5]

| | Weight ratio | | |
|---|---|---|---|
| | PRT799 | [Emim][DMP] | DMAc |
| BLANK | - | 1 | 3 |
| Comparative Example 3 | 1 | 1 | - |
| Comparative Example 4 | 1 | - | 3 |
| Example 4 | 1 | 1 | 3 |

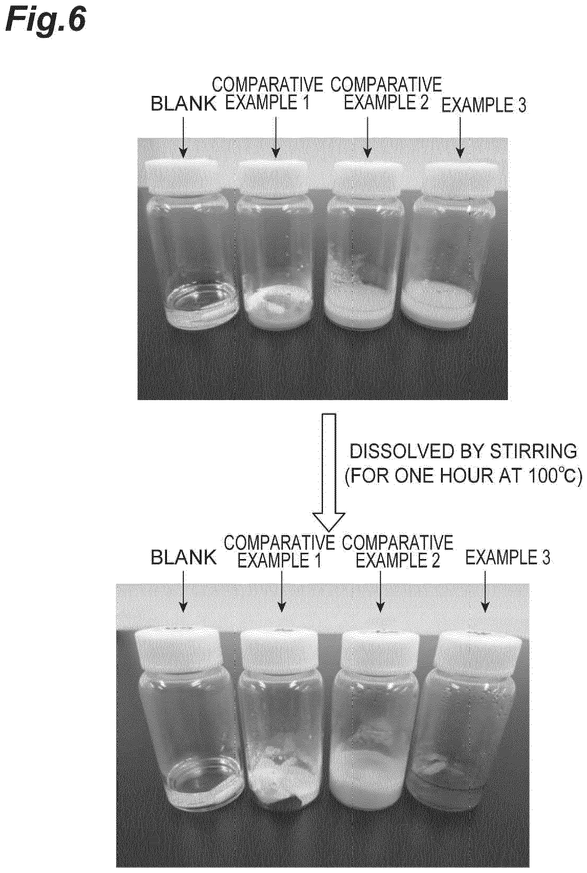

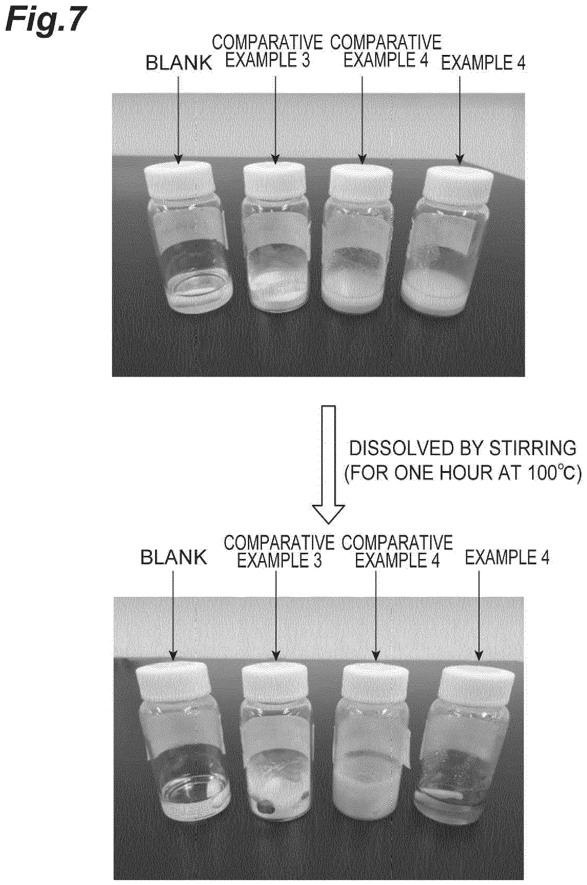

The results are shown in FIG. 6 and FIG. 7. FIG. 6 and FIG. 7 are images showing results obtained by observing the solubility of the spider silk fibroins of Examples 3 and 4 and Comparative Examples 1 to 4. As shown in FIG. 6 and FIG. 7, in Examples 3 and 4 including both the ionic liquid and the solvent, a protein solution in which the spider silk fibroin was completely dissolved was obtained regardless of the type of the ionic liquid and the solvent. On the other hand, in Comparative Examples 1 and 3 including only the ionic liquid and Comparative Examples 2 and 4 including only the solvent, the spider silk fibroin was not able to be dissolved.

[Test Example 4: Production and Evaluation of Protein Film]

<(4-1) Production of Protein Film>

Hexafluoro-2-propanol (HFIP) was added to the spider silk fibroin (PRT799) obtained in (1-1) of Test Example 1 and heated at 50°C for 3 hours with stirring, and the spider silk fibroin was dissolved. Then, 1-hexyl-3-methylimidazolium chloride [Hmim][Cl] was added as an ionic liquid to the obtained solution and mixed with inversion at 50°C for 1 hour to obtain a protein solution (composition: PRT799/[Hmim][Cl]/HFIP = 10/2.5/87.5 (mass ratio)).

0.5 mL of the protein solution was cast on a polytetrafluoroethylene (PTFE) film so that the thickness was 200 $\mu$m, and the film was heated at 60°C in a vacuum for 48 hours after 1 hour at room temperature and atmospheric pressure, and dried and desolvated to obtain a protein film A. The film thickness of the obtained protein film A was 30 to 60 $\mu$m.

For comparison, a protein film B was obtained in the same manner as above except that no ionic liquid was added.

<(4-2) Observation of form of protein film A>

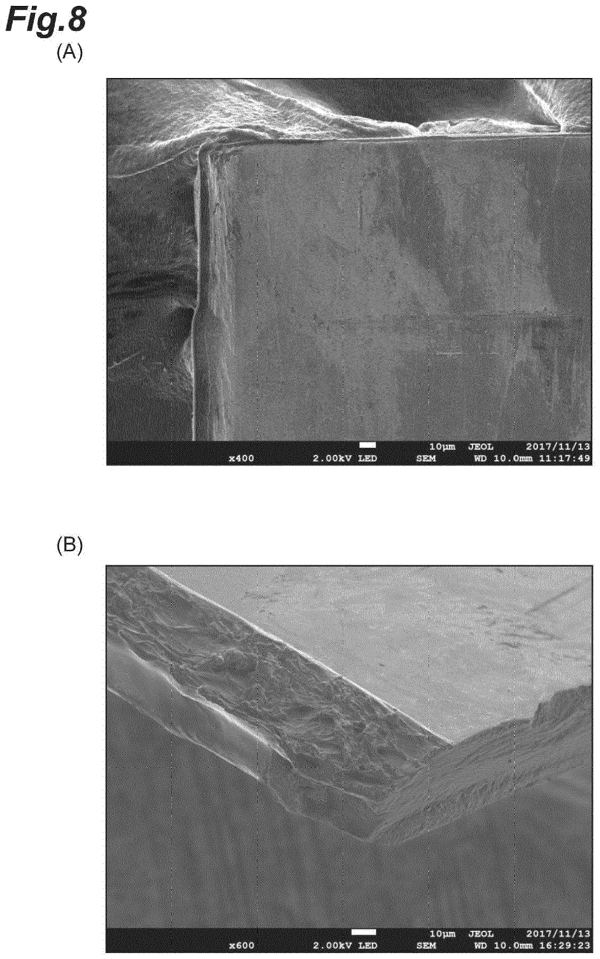

The form of the protein film A was observed using a scanning electron microscope (JSM-7100F, commercially available from JEOL Ltd.). The results are shown in FIG. 8. FIG. 8(A) is an image obtained by imaging the surface of the protein film A. FIG. 8(B) is an image obtained by imaging an end (edge) of the protein film A. As shown in FIG. 8, the entire protein film A was homogeneous.

<(4-3) Evaluation of content of ionic liquid in protein film A>

In the same manner as in (1-3) of Test Example 1, the cross section of the protein film A was imaged and elemental analysis was performed.

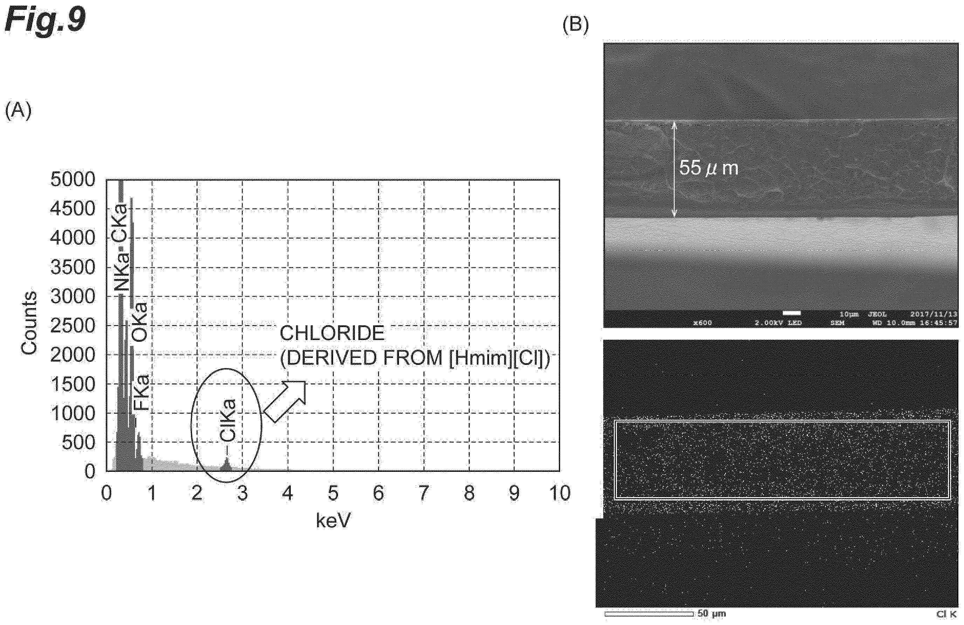

The results are shown in FIG. 9. FIG. 9(A) shows an EDX spectrum of a cross section of a protein film A. FIG. 9(B) is a diagram showing results of surface analysis (chloride abundance) according to EDX of the cross section of the protein film A. The EDX spectrum in FIG. 9(A) is measured in a region surrounded by a square in FIG. 9(B).

As shown in FIG. 9(A), in the EDX spectrum (point analysis) of the cross section of the protein film A, a peak was observed at 2.6 keV derived from chloride of [Hmim][Cl] used as the ionic liquid. In addition, as shown in FIG. 9(B), chloride was distributed in the protein film A. It can be understood that, since chloride was derived from the ionic liquid, the ionic liquid was dispersed and contained in the protein film A.

<(4-4) Evaluation of physical properties of protein film>

The protein film A and the protein film B were cut to a length of 10 mm to obtain test pieces. The test pieces were pulled in the lengthwise direction using a tensile testing machine 3342 (commercially available from Instron), and a tensile stress (vertical axis)-strain stress (horizontal axis) curve was measured. Test conditions were as follows; tensile speed: 10 mm/min, distance between gripping tools: 50 mm, temperature/humidity: uncontrolled, load cell capacity: 10 N, and the toughness was determined in the same manner as in (1-4) of Test Example 1.

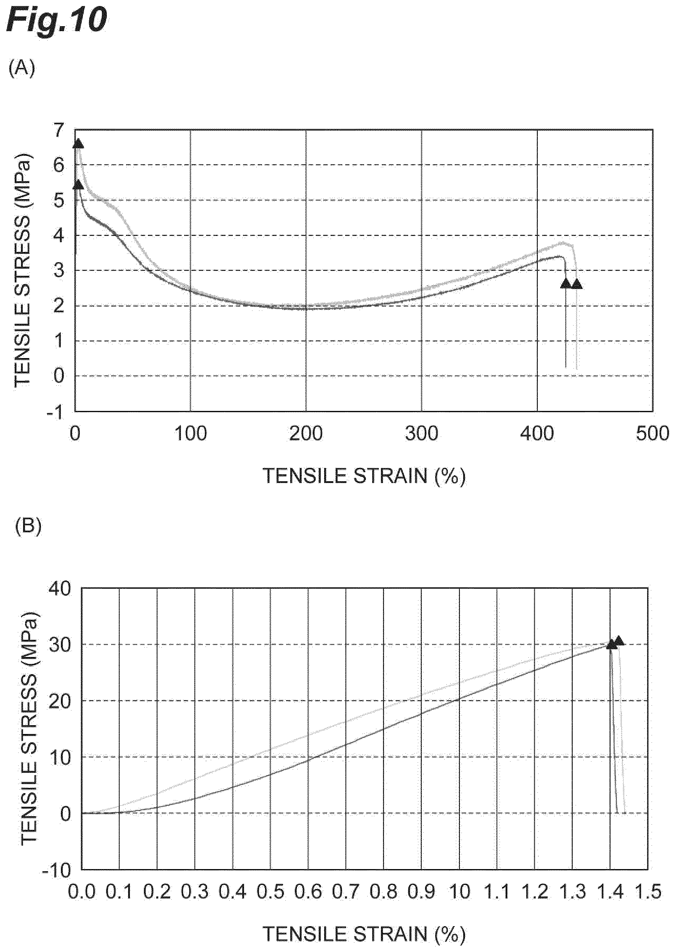

The results are shown in FIG. 10 and Table 6. FIG. 10(A) is a graph showing the results of the tensile test of the protein film A. FIG. 10(B) is a graph showing the results of the tensile test of the protein film B. In the graph of FIG. 10, the vertical axis represents the tensile stress (MPa), and the horizontal axis represents the tensile strain (%). Table 6 shows values of the tensile stress, the tensile strain, and the toughness determined from the results of the tensile test.

[Table 6]

| | Protein film A (with [Hmim][Cl]) | Protein film B (without [Hmim][Cl]) |
|---|---|---|
| Tensile stress (MPa) | 6.0 | 30.5 |
| Tensile strain (%) | 428.3 | 1.4 |
| Toughness (MJ/m$^3$) | 12 | 0.21 |

It can be understood that, compared to the protein film B in which no ionic liquid was added, in the protein film A in which the ionic liquid was added, the value of the tensile strain was significantly higher, and the ionic liquid plasticized the protein film. In addition, it can be understood that, compared to the protein film B in which no ionic liquid was added, in the protein film A in which the ionic liquid was added, the toughness was also improved, and the ionic liquid was included so that the elongation and toughness of the protein film increased.

**Reference Signs List**

1    Extrusion device
4    Drying device
6    Dope solution
10    Spinning device
20    Coagulation bathtub
21    Washing bathtub

## Claims

1. A protein molded article comprising a protein, wherein an ionic liquid is included inside the protein molded article.

2. The protein molded article according to claim 1, wherein the ionic liquid is dispersed inside the protein molded article.

3. The protein molded article according to claim 1 or 2, wherein the protein is a structural protein.

4. The protein molded article according to claim 3, wherein the structural protein is fibroin.

5. The protein molded article according to claim 4, wherein the fibroin is spider silk fibroin.

6. The protein molded article according to any one of claims 1 to 5, wherein the ionic liquid is a hydrophobic ionic liquid.

7. The protein molded article according to any one of claims 1 to 6, wherein the protein molded article is a fiber or a film.

8. The protein molded article according to any one of claims 1 to 7, wherein a temperature at which a heat weight loss ratio of the ionic liquid is 10% by mass is 200°C or higher.

9. A production method of the protein molded article according to any one of claims 1 to 8, comprising a step of adding the ionic liquid to a molding material of the protein molded article.

10. The production method according to claim 9, wherein the step of adding the ionic liquid is a step of causing infiltration of the ionic liquid into the molding material of the protein molded article.

11. The production method according to claim 9, wherein the step of adding the ionic liquid is a step of mixing the molding material of the protein molded article and the ionic liquid.

12. The production method according to claim 11, wherein the molding material is a molding solution prepared by dissolving the protein in a solvent.

13. The production method according to claim 12, further comprising a step of obtaining the protein molded article by coagulating the molding solution to which the ionic liquid is mixed through contact with a coagulating liquid, wherein the coagulating liquid includes an ion-supplying substance for supplying an ion to the coagulating liquid.

14. The production method according to claim 13, wherein the ion-supplying substance is a substance capable of causing coagulation of the protein.

15. The production method of the protein molded article according to any one of claims 1 to 8, comprising a step of obtaining a molded article by molding the molding material of the protein molded article and then causing infiltration of the ionic liquid into the molded article.

16. A protein solution comprising a protein, an ionic liquid, and a solvent.

17. The protein solution according to claim 16, wherein a content of the ionic liquid is from 10 to 20% by mass on a basis of a whole amount of the protein solution.

18. The protein solution according to claim 16 or 17, wherein the protein is a structural protein.

19. The protein solution according to claim 18, wherein the structural protein is fibroin.

20. The protein solution according to claim 19, wherein the fibroin is spider silk fibroin.

21. A protein molded article plasticizer comprising an ionic liquid.

22. The protein molded article plasticizer according to claim 21, wherein the protein is a structural protein.

23. The protein molded article plasticizer according to claim 22, wherein the structural protein is fibroin.

24. The protein molded article plasticizer according to claim 23, wherein the fibroin is spider silk fibroin.

EP 3 632 988 A1

## Fig.1

# *Fig.2*

(A)

(B)

*Fig.3*

PROPORTION OF PHOSPHORUS
(DERIVED FROM [Emim][DMP])
WITH RESPECT TO CARBON
$*: P < 0.05, n = 12$

# Fig.4

(A)

EXAMPLE 1

(B)

EXAMPLE 2

## Fig.5

(A)

(B)

# Fig.6

## Fig.7

# Fig.8

(A)

(B)

Fig.9

## Fig.10

(A)

(B)

## INTERNATIONAL SEARCH REPORT

International application No.

PCT/JP2018/021051

### A. CLASSIFICATION OF SUBJECT MATTER

Int.Cl. C08L89/00(2006.01)i, C08K5/529(2006.01)i, D01F4/02(2006.01)i, C12N15/00(2006.01)n

According to International Patent Classification (IPC) or to both national classification and IPC

### B. FIELDS SEARCHED

Minimum documentation searched (classification system followed by classification symbols)
Int.Cl. C08L1/00-101/14, C08K3/00-13/08, D01F1/00-6/96, 9/00-9/04, C12N15/00-15/90

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>A | WO 2016/067189 A1 (SILK BIOMATERIALS S. R. L.) 06 May 2016, claim 1, page 1, line 5 to page 2, line 22, page 19, line 16 to page 20, table 1, example 3 & JP 2017-533750 A, claim 1, paragraphs [0001]-[0005], [0073]-[0077], table 1, example 3 & US 2017/0312387 A1 & EP 3212246 A1 & CN 107073173 A & CA 2965600 A1 | 1-11, 15<br>12-14, 16-24 |
| X<br>A | JP 2010-111707 A (NISSHINBO HOLDINGS INC.) 20 May 2010, claim 12, paragraphs [0027], [0031], [0038]-[0039] & WO 2008/102747 A1 | 16-20<br>1-15, 21-24 |

☒ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 08 August 2018 (08.08.2018) | 21 August 2018 (21.08.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2018/021051 |

C (Continuation).    DOCUMENTS CONSIDERED TO BE RELEVANT

| Category* | Citation of document, with indication, where appropriate, of the relevant passages | Relevant to claim No. |
| --- | --- | --- |
| X<br>A | JP 2009-074073 A (TOHOKU UNIVERSITY) 09 April 2009, claims 1-2, paragraphs [0015], [0018] (Family: none) | 16, 18<br>1-15, 17, 19-24 |
| X<br>A | JP 2005-532440 A (DEGUSSA AG.) 27 October 2005, claims 1, 3, 24, paragraphs [0074]-[0077], table 1, examples & US 2006/0100323 A1, claims 1, 3, 24, paragraphs [0066]-[0076], table 1, examples & WO 2004/005391 A1 & EP 1519988 A1 & CA 2491587 A1 | 21<br>1-20, 22-24 |
| A | JP 2009-520846 A (BASF SE) 28 May 2009, entire text & JP 5371441 B2 & US 2008/0269477 A1, entire text & WO 2007/076979 A1 & EP 1966284 B1 & CA 2633623 A1 & KR 10-2008-0090451 A & CN 101346416 A | 1-24 |
| A | WO 2005/103158 A1 (NIPPON SHINYAKU CO., LTD.) 03 November 2005, entire text (Family: none) | 1-24 |

Form PCT/ISA/210 (continuation of second sheet) (January 2015)

**EP 3 632 988 A1**

**REFERENCES CITED IN THE DESCRIPTION**

**Patent documents cited in the description**

- WO 2005103158 A

- JP 2002238569 A

**Non-patent literature cited in the description**

- *Proc. Natl. Acad. Sci. USA,* 1972, vol. 69, 2110

- Molecular Cloning. Cold Spring Harbor Laboratory, 1989

**Sequence Listing**

SEQUENCE LISTING

```
<110>  Spiber Inc.
       Institute of National Colleges of Technology

<120>  PROTEIN MOLDED ARTICLE AND METHOD FOR PRODUCING SAME,
       PROTEIN SOLUTION, AND PROTEIN MOLDED ARTICLE
       PLASTICIZER

<130>  MEH/93823EP1

<140>  PCT/JP2018/021051
<141>  2018-05-31

<150>  JP2017-109974
<151>  2017-06-02

<160>  8

<170>  PatentIn version 3.5

<210>  1
<211>  601
<212>  PRT
<213>  Artificial Sequence

<220>
<223>  PRT410

<400>  1

Met His His His His His His Ser Ser Gly Ser Ser Gly Pro Gly Gln
1               5                   10                  15


Gln Gly Pro Tyr Gly Pro Gly Ala Ser Ala Ala Ala Ala Ala Gly Gln
            20                  25                  30


Asn Gly Pro Gly Ser Gly Gln Gln Gly Pro Gly Gln Ser Gly Gln Tyr
            35                  40                  45


Gly Pro Gly Gln Gln Gly Pro Gly Gln Gln Gly Pro Gly Ser Ser Ala
        50                  55                  60


Ala Ala Ala Ala Gly Pro Gly Gln Tyr Gly Pro Gly Gln Gln Gly Pro
65                  70                  75                  80


Ser Ala Ser Ala Ala Ala Ala Ala Gly Pro Gly Ser Gly Gln Gln Gly
                85                  90                  95


Pro Gly Ala Ser Gly Gln Tyr Gly Pro Gly Gln Gln Gly Pro Gly Gln
            100                 105                 110


Gln Gly Pro Gly Ser Ser Ala Ala Ala Ala Ala Gly Gln Tyr Gly Ser
            115                 120                 125
```

Gly Pro Gly Gln Gln Gly Pro Tyr Gly Ser Ala Ala Ala Ala Ala Gly
    130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
                165                 170                 175

Ala Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
        195                 200                 205

Gln Gln Gly Pro Tyr Gly Pro Gly Gln Ser Gly Ser Gly Gln Gln Gly
    210                 215                 220

Pro Gly Gln Gln Gly Pro Tyr Ala Ser Ala Ala Ala Ala Ala Gly Pro
225                 230                 235                 240

Gly Gln Gln Gly Pro Tyr Gly Pro Gly Ser Ser Ala Ala Ala Ala Ala
            245                 250                 255

Gly Gln Tyr Gly Tyr Gly Pro Gly Gln Gln Gly Pro Tyr Gly Pro Gly
        260                 265                 270

Ala Ser Gly Gln Asn Gly Pro Gly Ser Gly Gln Tyr Gly Pro Gly Gln
        275                 280                 285

Gln Gly Pro Gly Gln Ser Ala Ala Ala Ala Gly Pro Gly Gln Gln
    290                 295                 300

Gly Pro Tyr Gly Pro Gly Ala Ser Ala Ala Ala Ala Gly Gln Tyr
305                 310                 315                 320

Gly Pro Gly Gln Gln Gly Pro Gly Gln Tyr Gly Pro Gly Ser Ser Gly
                325                 330                 335

Pro Gly Gln Gln Gly Pro Tyr Gly Pro Gly Ser Ser Ala Ala Ala Ala
            340                 345                 350

Ala Gly Gln Tyr Gly Pro Gly Gln Gln Gly Pro Tyr Gly Pro Gly Gln
        355                 360                 365

Ser Ala Ala Ala Ala Ala Gly Gln Tyr Gln Gln Gly Pro Gly Gln Gln
370                 375                 380

```
Gly Pro Tyr Gly Pro Gly Ala Ser Gly Pro Gly Gln Gln Gly Pro Tyr
385             390             395             400

Gly Pro Gly Ala Ser Ala Ala Ala Ala Ala Gly Pro Gly Gln Tyr Gly
                405             410             415

Pro Gly Gln Gln Gly Pro Ser Ala Ser Ala Ala Ala Ala Ala Gly Gln
            420             425             430

Tyr Gly Ser Gly Pro Gly Gln Tyr Gly Pro Tyr Gly Pro Gly Gln Ser
        435             440             445

Gly Pro Gly Ser Gly Gln Gln Gly Gln Gly Pro Tyr Gly Pro Gly Ala
    450             455             460

Ser Ala Ala Ala Ala Ala Gly Gln Tyr Gly Pro Gly Gln Gln Gly Pro
465             470             475             480

Tyr Gly Pro Gly Gln Ser Ala Ala Ala Ala Gly Pro Gly Ser Gly
            485             490             495

Gln Tyr Gly Pro Gly Ala Ser Gly Gln Asn Gly Pro Gly Ser Gly Gln
        500             505             510

Tyr Gly Pro Gly Gln Gln Gly Pro Gly Gln Ser Ala Ala Ala Ala Ala
    515             520             525

Gly Gln Tyr Gln Gln Gly Pro Gly Gln Gln Gly Pro Tyr Gly Pro Gly
    530             535             540

Ala Ser Ala Ala Ala Ala Ala Gly Gln Tyr Gly Ser Gly Pro Gly Gln
545             550             555             560

Gln Gly Pro Tyr Gly Pro Gly Gln Ser Gly Ser Gly Gln Gln Gly Pro
            565             570             575

Gly Gln Gln Gly Pro Tyr Ala Ser Ala Ala Ala Ala Ala Gly Pro Gly
        580             585             590

Ser Gly Gln Gln Gly Pro Gly Ala Ser
    595             600
```

```
<210>   2
<211>   559
<212>   PRT
<213>   Artificial Sequence

<220>
```

<223>  Recombinant spider silk protein Flag_92_short2

<400>  2

Met His His His His His His His His His His Ser Ser Gly Ser Ser
1                   5                   10                  15

Leu Glu Val Leu Phe Gln Gly Pro Gly Ala Gly Gly Ser Gly Pro Gly
            20                  25                  30

Gly Ala Gly Pro Gly Gly Val Gly Pro Gly Gly Ser Gly Pro Gly Gly
            35                  40                  45

Val Gly Pro Gly Gly Ser Gly Pro Gly Gly Val Gly Pro Gly Gly Ser
    50                  55                  60

Gly Pro Gly Gly Val Gly Pro Gly Gly Ala Gly Gly Pro Tyr Gly Pro
65                  70                  75                  80

Gly Gly Ser Gly Pro Gly Gly Ala Gly Gly Ala Gly Gly Pro Gly Gly
                85                  90                  95

Ala Tyr Gly Pro Gly Gly Ser Tyr Gly Pro Gly Gly Ser Gly Gly Pro
            100                 105                 110

Gly Gly Ala Gly Gly Pro Tyr Gly Pro Gly Gly Glu Gly Pro Gly Gly
            115                 120                 125

Ala Gly Gly Pro Tyr Gly Pro Gly Gly Ala Gly Gly Pro Tyr Gly Pro
            130                 135                 140

Gly Gly Ala Gly Gly Pro Tyr Gly Pro Gly Gly Glu Gly Gly Pro Tyr
145                 150                 155                 160

Gly Pro Gly Gly Ser Tyr Gly Pro Gly Gly Ala Gly Gly Pro Tyr Gly
                165                 170                 175

Pro Gly Gly Pro Tyr Gly Pro Gly Gly Glu Gly Pro Gly Gly Ala Gly
            180                 185                 190

Gly Pro Tyr Gly Pro Gly Gly Val Gly Pro Gly Gly Gly Pro Gly
            195                 200                 205

Gly Tyr Gly Pro Gly Gly Ala Gly Pro Gly Gly Tyr Gly Pro Gly Gly
    210                 215                 220

Ser Gly Pro Gly Gly Tyr Gly Pro Gly Gly Ser Gly Pro Gly Gly Tyr
225                 230                 235                 240

Gly Pro Gly Gly Ser Gly Pro Gly Gly Tyr Gly Pro Gly Gly Ser Gly
        245             250             255

Pro Gly Gly Tyr Gly Pro Gly Gly Ser Gly Pro Gly Gly Ser Gly Pro
        260             265             270

Gly Gly Tyr Gly Pro Gly Gly Ser Gly Pro Gly Gly Ser Gly Pro Gly
        275             280             285

Gly Tyr Gly Pro Gly Gly Ser Gly Pro Gly Gly Tyr Gly Pro Gly Gly
        290             295             300

Ser Gly Pro Gly Gly Ser Gly Pro Gly Gly Tyr Gly Pro Gly Gly Ser
305             310             315             320

Gly Pro Gly Gly Ser Gly Pro Gly Gly Tyr Gly Pro Gly Gly Ser Gly
        325             330             335

Pro Gly Gly Phe Gly Pro Gly Gly Phe Gly Pro Gly Gly Ser Gly Pro
        340             345             350

Gly Gly Tyr Gly Pro Gly Gly Ser Gly Pro Gly Gly Ala Gly Pro Gly
        355             360             365

Gly Val Gly Pro Gly Gly Phe Gly Pro Gly Gly Ala Gly Pro Gly Gly
        370             375             380

Ala Gly Pro Gly Gly Ala Gly Pro Gly Gly Ala Gly Pro Gly Gly Ala
385             390             395             400

Gly Pro Gly Gly Ala Gly Pro Gly Gly Ala Gly Pro Gly Gly Ala Gly
        405             410             415

Pro Gly Gly Ala Gly Gly Ala Gly Gly Ala Gly Gly Ala Gly Gly Ser
        420             425             430

Gly Gly Ala Gly Gly Ser Gly Gly Thr Thr Ile Ile Glu Asp Leu Asp
        435             440             445

Ile Thr Ile Asp Gly Ala Asp Gly Pro Ile Thr Ile Ser Glu Glu Leu
        450             455             460

Thr Ile Ser Ala Tyr Tyr Pro Ser Ser Arg Val Pro Asp Met Val Asn
465             470             475             480

Gly Ile Met Ser Ala Met Gln Gly Ser Gly Phe Asn Tyr Gln Met Phe
        485             490             495

23

```
Gly Asn Met Leu Ser Gln Tyr Ser Ser Gly Ser Gly Thr Cys Asn Pro
            500                 505             510

Asn Asn Val Asn Val Leu Met Asp Ala Leu Leu Ala Ala Leu His Cys
            515                 520             525

Leu Ser Asn His Gly Ser Ser Ser Phe Ala Pro Ser Pro Thr Pro Ala
            530                 535             540

Ala Met Ser Ala Tyr Ser Asn Ser Val Gly Arg Met Phe Ala Tyr
545                 550             555
```

```
<210>  3
<211>  252
<212>  PRT
<213>  Artificial Sequence

<220>
<223>  Collagen-type4-Kai

<400>  3
```

```
Met His His His His His His Ser Ser Gly Ser Ser Lys Asp Gly Val
1               5               10              15

Pro Gly Phe Pro Gly Ser Glu Gly Val Lys Gly Asn Arg Gly Phe Pro
            20                  25              30

Gly Leu Met Gly Glu Asp Gly Ile Lys Gly Gln Lys Gly Asp Ile Gly
            35                  40              45

Pro Pro Gly Phe Arg Gly Pro Thr Glu Tyr Tyr Asp Thr Tyr Gln Glu
            50                  55              60

Lys Gly Asp Glu Gly Thr Pro Gly Pro Pro Gly Pro Arg Gly Ala Arg
65                  70              75                  80

Gly Pro Gln Gly Pro Ser Gly Pro Pro Gly Val Pro Gly Ser Pro Gly
                    85                  90              95

Ser Ser Arg Pro Gly Leu Arg Gly Ala Pro Gly Trp Pro Gly Leu Lys
            100                 105             110

Gly Ser Lys Gly Glu Arg Gly Arg Pro Gly Lys Asp Ala Met Gly Thr
            115                 120             125

Pro Gly Ser Pro Gly Cys Ala Gly Ser Pro Gly Leu Pro Gly Ser Pro
            130                 135             140
```

```
Gly Pro Pro Gly Pro Pro Gly Asp Ile Val Phe Arg Lys Gly Pro Pro
145             150             155             160

Gly Asp His Gly Leu Pro Gly Tyr Leu Gly Ser Pro Gly Ile Pro Gly
                165             170             175

Val Asp Gly Pro Lys Gly Glu Pro Gly Leu Leu Cys Thr Gln Cys Pro
                180             185             190

Tyr Ile Pro Gly Pro Pro Gly Leu Pro Gly Leu Pro Gly Leu His Gly
            195             200             205

Val Lys Gly Ile Pro Gly Arg Gln Gly Ala Ala Gly Leu Lys Gly Ser
    210             215             220

Pro Gly Ser Pro Gly Asn Thr Gly Leu Pro Gly Phe Pro Gly Phe Pro
225             230             235             240

Gly Ala Gln Gly Asp Pro Gly Leu Lys Gly Glu Lys
                245             250
```

```
<210> 4
<211> 310
<212> PRT
<213> Artificial Sequence

<220>
<223> Resilin-Kai

<400> 4
```

```
Met His His His His His His Pro Glu Pro Pro Val Asn Ser Tyr Leu
1               5               10              15

Pro Pro Ser Asp Ser Tyr Gly Ala Pro Gly Gln Ser Gly Pro Gly Gly
            20              25              30

Arg Pro Ser Asp Ser Tyr Gly Ala Pro Gly Gly Gly Asn Gly Gly Arg
        35              40              45

Pro Ser Asp Ser Tyr Gly Ala Pro Gly Gln Gly Gln Gly Gln Gly Gln
    50              55              60

Gly Gln Gly Gly Tyr Ala Gly Lys Pro Ser Asp Ser Tyr Gly Ala Pro
65              70              75              80

Gly Gly Gly Asp Gly Asn Gly Gly Arg Pro Ser Ser Ser Tyr Gly Ala
                85              90              95
```

```
Pro Gly Gly Gly Asn Gly Gly Arg Pro Ser Asp Thr Tyr Gly Ala Pro
            100                 105                 110

Gly Gly Gly Asn Gly Gly Arg Pro Ser Asp Thr Tyr Gly Ala Pro Gly
            115                 120                 125

Gly Gly Gly Asn Gly Asn Gly Gly Arg Pro Ser Ser Ser Tyr Gly Ala
            130                 135                 140

Pro Gly Gln Gly Gln Gly Asn Gly Asn Gly Gly Arg Pro Ser Ser Ser
145                 150                 155                 160

Tyr Gly Ala Pro Gly Gly Gly Asn Gly Gly Arg Pro Ser Asp Thr Tyr
                165                 170                 175

Gly Ala Pro Gly Gly Gly Asn Gly Gly Arg Pro Ser Asp Thr Tyr Gly
                180                 185                 190

Ala Pro Gly Gly Gly Asn Asn Gly Gly Arg Pro Ser Ser Ser Tyr Gly
                195                 200                 205

Ala Pro Gly Gly Gly Asn Gly Gly Arg Pro Ser Asp Thr Tyr Gly Ala
210                 215                 220

Pro Gly Gly Gly Asn Gly Asn Gly Ser Gly Gly Arg Pro Ser Ser Ser
225                 230                 235                 240

Tyr Gly Ala Pro Gly Gln Gly Gln Gly Gly Phe Gly Gly Arg Pro Ser
                245                 250                 255

Asp Ser Tyr Gly Ala Pro Gly Gln Asn Gln Lys Pro Ser Asp Ser Tyr
                260                 265                 270

Gly Ala Pro Gly Ser Gly Asn Gly Asn Gly Gly Arg Pro Ser Ser Ser
                275                 280                 285

Tyr Gly Ala Pro Gly Ser Gly Pro Gly Gly Arg Pro Ser Asp Ser Tyr
290                 295                 300

Gly Pro Pro Ala Ser Gly
305                 310


<210>   5
<211>   282
<212>   PRT
<213>   Artificial Sequence

<220>
<223>   elastin short
```

<400> 5

Met His His His His His Ser Ser Gly Ser Ser Leu Gly Val Ser
1               5               10              15

Ala Gly Ala Val Val Pro Gln Pro Gly Ala Gly Val Lys Pro Gly Lys
        20              25              30

Val Pro Gly Val Gly Leu Pro Gly Val Tyr Pro Gly Gly Val Leu Pro
        35              40              45

Gly Ala Arg Phe Pro Gly Val Gly Val Leu Pro Gly Val Pro Thr Gly
    50              55              60

Ala Gly Val Lys Pro Lys Ala Pro Gly Val Gly Gly Ala Phe Ala Gly
65              70              75              80

Ile Pro Gly Val Gly Pro Phe Gly Gly Pro Gln Pro Gly Val Pro Leu
            85              90              95

Gly Tyr Pro Ile Lys Ala Pro Lys Leu Pro Gly Gly Tyr Gly Leu Pro
            100             105             110

Tyr Thr Thr Gly Lys Leu Pro Tyr Gly Tyr Gly Pro Gly Gly Val Ala
        115             120             125

Gly Ala Ala Gly Lys Ala Gly Tyr Pro Thr Gly Thr Gly Val Gly Pro
    130             135             140

Gln Ala Ala Ala Ala Ala Ala Ala Lys Ala Ala Ala Lys Phe Gly Ala
145             150             155             160

Gly Ala Ala Gly Val Leu Pro Gly Val Gly Gly Ala Gly Val Pro Gly
            165             170             175

Val Pro Gly Ala Ile Pro Gly Ile Gly Ile Ala Gly Val Gly Thr
            180             185             190

Pro Ala Ala Ala Ala Ala Ala Ala Ala Ala Lys Ala Ala Lys Tyr
    195             200             205

Gly Ala Ala Ala Gly Leu Val Pro Gly Gly Pro Gly Phe Gly Pro Gly
    210             215             220

Val Val Gly Val Pro Gly Ala Gly Val Pro Gly Val Gly Val Pro Gly
225             230             235             240

```
        Ala Gly Ile Pro Val Val Pro Gly Ala Gly Ile Pro Gly Ala Ala Val
                        245             250             255

        Pro Gly Val Val Ser Pro Glu Ala Ala Ala Lys Ala Ala Ala Lys Ala
                        260             265             270

        Ala Lys Tyr Gly Ala Arg Pro Gly Val Gly
                275             280
```

```
<210>  6
<211>  468
<212>  PRT
<213>  Artificial Sequence

<220>
<223>  type I keratin 26

<400>  6

        Met Ser Phe Arg Leu Ser Gly Val Ser Arg Arg Leu Cys Ser Gln Ala
        1               5               10              15

        Gly Thr Gly Arg Leu Thr Gly Gly Arg Thr Gly Phe Arg Ala Gly Asn
                        20              25              30

        Val Cys Ser Gly Leu Gly Ala Gly Ser Ser Phe Ser Gly Pro Leu Gly
                35              40              45

        Ser Val Ser Ser Lys Gly Ser Phe Ser His Gly Gly Gly Gly Leu Gly
                50              55              60

        Ser Gly Val Cys Thr Gly Phe Leu Glu Asn Glu His Gly Leu Leu Pro
        65              70              75              80

        Gly Asn Glu Lys Val Thr Leu Gln Asn Leu Asn Asp Arg Leu Ala Ser
                        85              90              95

        Tyr Leu Asp His Val Cys Thr Leu Glu Glu Ala Asn Ala Asp Leu Glu
                        100             105             110

        Gln Lys Ile Lys Gly Trp Tyr Glu Lys Tyr Gly Pro Gly Ser Gly Arg
                        115             120             125

        Gln Leu Ala His Asp Tyr Ser Lys Tyr Phe Ser Val Thr Glu Asp Leu
                130             135             140

        Lys Arg Gln Ile Ile Ser Val Thr Thr Cys Asn Ala Ser Ile Val Leu
        145             150             155             160

        Gln Asn Glu Asn Ala Arg Leu Thr Ala Asp Asp Phe Arg Leu Lys Cys
```

```
                    165                    170                    175

Glu Asn Glu Leu Ala Leu His Gln Ser Val Glu Ala Asp Ile Asn Gly
            180                    185                    190

Leu His Arg Val Met Asp Glu Leu Thr Leu Cys Thr Ser Asp Leu Glu
            195                    200                    205

Met Gln Cys Glu Ala Leu Ser Glu Glu Leu Thr Tyr Leu Lys Lys Asn
            210                    215                    220

His Gln Glu Glu Met Lys Val Met Gln Gly Ala Ala Arg Gly Asn Val
225                    230                    235                    240

Asn Val Glu Ile Asn Ala Ala Pro Gly Val Asp Leu Thr Val Leu Leu
            245                    250                    255

Asn Asn Met Arg Ala Glu Tyr Glu Asp Leu Ala Glu Gln Asn His Glu
            260                    265                    270

Asp Ala Glu Ala Trp Phe Ser Glu Lys Ser Thr Ser Leu His Gln Gln
            275                    280                    285

Ile Ser Asp Asp Ala Gly Ala Ala Met Ala Ala Arg Asn Glu Leu Met
    290                    295                    300

Glu Leu Lys Arg Asn Leu Gln Thr Leu Glu Ile Glu Leu Gln Ser Leu
305                    310                    315                    320

Leu Ala Met Lys His Ser Tyr Glu Cys Ser Leu Ala Glu Thr Glu Ser
            325                    330                    335

Asn Tyr Cys His Gln Leu Gln Gln Ile Gln Glu Gln Ile Gly Ala Met
            340                    345                    350

Glu Asp Gln Leu Gln Gln Ile Arg Met Glu Thr Glu Gly Gln Lys Leu
            355                    360                    365

Glu His Glu Arg Leu Leu Asp Val Lys Ile Phe Leu Glu Lys Glu Ile
            370                    375                    380

Glu Met Tyr Cys Lys Leu Ile Asp Gly Glu Gly Arg Lys Ser Lys Ser
385                    390                    395                    400

Thr Cys Tyr Lys Ser Glu Gly Arg Gly Pro Lys Asn Ser Glu Asn Gln
            405                    410                    415
```

```
         Val Lys Asp Ser Lys Glu Glu Ala Val Val Lys Thr Val Val Gly Glu
                     420                 425                 430


         Leu Asp Gln Leu Gly Ser Val Leu Ser Leu Arg Val His Ser Val Glu
                     435                 440                 445


         Glu Lys Ser Ser Lys Ile Ser Asn Ile Thr Met Glu Gln Arg Leu Pro
                     450                 455                 460


         Ser Lys Val Pro
         465



         <210>   7
         <211>   12
         <212>   PRT
         <213>   Artificial Sequence

         <220>
         <223>   His Tag

         <400>   7

         Met His His His His His His Ser Ser Gly Ser Ser
         1                   5                   10



         <210>   8
         <211>   2375
         <212>   PRT
         <213>   Artificial Sequence

         <220>
         <223>   PRT799

         <400>   8

         Met His His His His His His Ser Ser Gly Ser Ser Gly Pro Gly Gln
         1                   5                   10                  15


         Gln Gly Pro Tyr Gly Pro Gly Ala Ser Ala Ala Ala Ala Gly Gln
                         20                  25                  30


         Asn Gly Pro Gly Ser Gly Gln Gln Gly Pro Gly Gln Ser Gly Gln Tyr
                     35                  40                  45


         Gly Pro Gly Gln Gln Gly Pro Gly Gln Gln Gly Pro Gly Ser Ser Ala
                     50                  55                  60


         Ala Ala Ala Ala Gly Pro Gly Gln Tyr Gly Pro Gly Gln Gln Gly Pro
         65                  70                  75                  80


         Ser Ala Ser Ala Ala Ala Ala Ala Gly Pro Gly Ser Gly Gln Gln Gly
                         85                  90                  95
```

30

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Gly Gln Tyr Gly Ser
            115                 120                 125

Gly Pro Gly Gln Gln Gly Pro Tyr Gly Ser Ala Ala Ala Ala Gly
            130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
                165                 170                 175

Ala Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
            195                 200                 205

Gln Gln Gly Pro Tyr Gly Pro Gly Gln Ser Gly Ser Gly Gln Gln Gly
            210                 215                 220

Pro Gly Gln Gln Gly Pro Tyr Ala Ser Ala Ala Ala Ala Ala Gly Pro
225                 230                 235                 240

Gly Gln Gln Gly Pro Tyr Gly Pro Gly Ser Ser Ala Ala Ala Ala Ala
                245                 250                 255

Gly Gln Tyr Gly Tyr Gly Pro Gly Gln Gln Gly Pro Tyr Gly Pro Gly
            260                 265                 270

Ala Ser Gly Gln Asn Gly Pro Gly Ser Gly Gln Tyr Gly Pro Gly Gln
            275                 280                 285

Gln Gly Pro Gly Gln Ser Ala Ala Ala Ala Gly Pro Gly Gln Gln
            290                 295                 300

Gly Pro Tyr Gly Pro Gly Ala Ser Ala Ala Ala Ala Gly Gln Tyr
305                 310                 315                 320

Gly Pro Gly Gln Gln Gly Pro Gly Gln Tyr Gly Pro Gly Ser Ser Gly
                325                 330                 335

Pro Gly Gln Gln Gly Pro Tyr Gly Pro Gly Ser Ser Ala Ala Ala Ala
            340                 345                 350

Ala Gly Gln Tyr Gly Pro Gly Gln Gln Gly Pro Tyr Gly Pro Gly Gln
        355                     360                 365

Ser Ala Ala Ala Ala Ala Gly Gln Tyr Gln Gln Gly Pro Gly Gln Gln
        370                     375                 380

Gly Pro Tyr Gly Pro Gly Ala Ser Gly Pro Gly Gln Gln Gly Pro Tyr
385                     390                 395                     400

Gly Pro Gly Ala Ser Ala Ala Ala Ala Ala Gly Pro Gly Gln Tyr Gly
                405                     410                 415

Pro Gly Gln Gln Gly Pro Ser Ala Ser Ala Ala Ala Ala Gly Gln
            420                     425                 430

Tyr Gly Ser Gly Pro Gly Gln Tyr Gly Pro Tyr Gly Pro Gly Gln Ser
            435                     440                 445

Gly Pro Gly Ser Gly Gln Gln Gly Gln Gly Pro Tyr Gly Pro Gly Ala
        450                     455                 460

Ser Ala Ala Ala Ala Ala Gly Gln Tyr Gly Pro Gly Gln Gln Gly Pro
465                     470                     475                     480

Tyr Gly Pro Gly Gln Ser Ala Ala Ala Ala Ala Gly Pro Gly Ser Gly
                485                     490                 495

Gln Tyr Gly Pro Gly Ala Ser Gly Gln Asn Gly Pro Gly Ser Gly Gln
        500                     505                 510

Tyr Gly Pro Gly Gln Gln Gly Pro Gly Gln Ser Ala Ala Ala Ala Ala
        515                     520                 525

Gly Gln Tyr Gln Gln Gly Pro Gly Gln Gln Gly Pro Tyr Gly Pro Gly
        530                     535                 540

Ala Ser Ala Ala Ala Ala Ala Gly Gln Tyr Gly Ser Gly Pro Gly Gln
545                     550                     555                     560

Gln Gly Pro Tyr Gly Pro Gly Gln Ser Gly Ser Gly Gln Gln Gly Pro
                565                     570                 575

Gly Gln Gln Gly Pro Tyr Ala Ser Ala Ala Ala Ala Gly Pro Gly
        580                     585                 590

Ser Gly Gln Gln Gly Pro Gly Ala Ser Gly Gln Gln Gly Pro Tyr Gly

595                           600                           605

Pro Gly Ala Ser Ala Ala Ala Ala Ala Gly Gln Asn Gly Pro Gly Ser
        610                 615                 620

Gly Gln Gln Gly Pro Gly Gln Ser Gly Gln Tyr Gly Pro Gly Gln Gln
625                 630                 635                 640

Gly Pro Gly Gln Gln Gly Pro Gly Ser Ser Ala Ala Ala Ala Gly
                645                 650                 655

Pro Gly Gln Tyr Gly Pro Gly Gln Gln Gly Pro Ser Ala Ser Ala Ala
            660                 665                 670

Ala Ala Ala Gly Pro Gly Ser Gly Gln Gln Gly Pro Gly Ala Ser Gly
        675                 680                 685

Gln Tyr Gly Pro Gly Gln Gln Gly Pro Gly Gln Gln Gly Pro Gly Ser
    690                 695                 700

Ser Ala Ala Ala Ala Ala Gly Gln Tyr Gly Ser Gly Pro Gly Gln Gln
705                 710                 715                 720

Gly Pro Tyr Gly Ser Ala Ala Ala Ala Gly Pro Gly Ser Gly Gln
            725                 730                 735

Tyr Gly Gln Gly Pro Tyr Gly Pro Gly Ala Ser Gly Pro Gly Gln Tyr
        740                 745                 750

Gly Pro Gly Gln Gln Gly Pro Ser Ala Ser Ala Ala Ala Ala Ala Gly
        755                 760                 765

Ser Gly Gln Gln Gly Pro Gly Gln Tyr Gly Pro Tyr Ala Ser Ala Ala
    770                 775                 780

Ala Ala Ala Gly Gln Tyr Gly Ser Gly Pro Gly Gln Gln Gly Pro Tyr
785                 790                 795                 800

Gly Pro Gly Gln Ser Gly Ser Gly Gln Gln Gly Pro Gly Gln Gln Gly
            805                 810                 815

Pro Tyr Ala Ser Ala Ala Ala Ala Ala Gly Pro Gly Gln Gln Gly Pro
        820                 825                 830

Tyr Gly Pro Gly Ser Ser Ala Ala Ala Ala Gly Gln Tyr Gly Tyr
    835                 840                 845

Gly Pro Gly Gln Gln Gly Pro Tyr Gly Pro Gly Ala Ser Gly Gln Asn
850                          855                  860

Gly Pro Gly Ser Gly Gln Tyr Gly Pro Gly Gln Gln Gly Pro Gly Gln
865                  870                  875                  880

Ser Ala Ala Ala Ala Ala Gly Pro Gly Gln Gln Gly Pro Tyr Gly Pro
              885                  890                  895

Gly Ala Ser Ala Ala Ala Ala Ala Gly Gln Tyr Gly Pro Gly Gln Gln
         900                  905                  910

Gly Pro Gly Gln Tyr Gly Pro Gly Ser Ser Gly Pro Gly Gln Gln Gly
         915                  920                  925

Pro Tyr Gly Pro Gly Ser Ser Ala Ala Ala Ala Ala Gly Gln Tyr Gly
    930                  935                  940

Pro Gly Gln Gln Gly Pro Tyr Gly Pro Gly Gln Ser Ala Ala Ala Ala
945                  950                  955                  960

Ala Gly Gln Tyr Gln Gln Gly Pro Gly Gln Gln Gly Pro Tyr Gly Pro
              965                  970                  975

Gly Ala Ser Gly Pro Gly Gln Gln Gly Pro Tyr Gly Pro Gly Ala Ser
         980                  985                  990

Ala Ala Ala Ala Ala Gly Pro Gly Gln Tyr Gly Pro Gly Gln Gln Gly
         995                  1000                 1005

Pro Ser Ala Ser Ala Ala Ala Ala Ala Gly Gln Tyr Gly Ser Gly
    1010                 1015                 1020

Pro Gly Gln Tyr Gly Pro Tyr Gly Pro Gly Gln Ser Gly Pro Gly
    1025                 1030                 1035

Ser Gly Gln Gln Gly Gln Gly Pro Tyr Gly Pro Gly Ala Ser Ala
    1040                 1045                 1050

Ala Ala Ala Ala Gly Gln Tyr Gly Pro Gly Gln Gln Gly Pro Tyr
    1055                 1060                 1065

Gly Pro Gly Gln Ser Ala Ala Ala Ala Ala Gly Pro Gly Ser Gly
    1070                 1075                 1080

Gln Tyr Gly Pro Gly Ala Ser Gly Gln Asn Gly Pro Gly Ser Gly
    1085                 1090                 1095

```
Gln Tyr  Gly Pro Gly Gln Gln  Gly Pro Gly Gln Ser  Ala Ala Ala
    1100             1105              1110

Ala Ala  Gly Gln Tyr Gln Gln  Gly Pro Gly Gln Gln  Gly Pro Tyr
    1115             1120              1125

Gly Pro  Gly Ala Ser Ala Ala  Ala Ala Ala Gly Gln  Tyr Gly Ser
    1130             1135              1140

Gly Pro  Gly Gln Gln Gly Pro  Tyr Gly Pro Gly Gln  Ser Gly Ser
    1145             1150              1155

Gly Gln  Gln Gly Pro Gly Gln  Gln Gly Pro Tyr Ala  Ser Ala Ala
    1160             1165              1170

Ala Ala  Ala Gly Pro Gly Ser  Gly Gln Gln Gly Pro  Gly Ala Ser
    1175             1180              1185

Gly Gln  Gln Gly Pro Tyr Gly  Pro Gly Ala Ser Ala  Ala Ala Ala
    1190             1195              1200

Ala Gly  Gln Asn Gly Pro Gly  Ser Gly Gln Gln Gly  Pro Gly Gln
    1205             1210              1215

Ser Gly  Gln Tyr Gly Pro Gly  Gln Gln Gly Pro Gly  Gln Gln Gly
    1220             1225              1230

Pro Gly  Ser Ser Ala Ala Ala  Ala Ala Gly Pro Gly  Gln Tyr Gly
    1235             1240              1245

Pro Gly  Gln Gln Gly Pro Ser  Ala Ser Ala Ala Ala  Ala Ala Gly
    1250             1255              1260

Pro Gly  Ser Gly Gln Gln Gly  Pro Gly Ala Ser Gly  Gln Tyr Gly
    1265             1270              1275

Pro Gly  Gln Gln Gly Pro Gly  Gln Gln Gly Pro Gly  Ser Ser Ala
    1280             1285              1290

Ala Ala  Ala Ala Gly Gln Tyr  Gly Ser Gly Pro Gly  Gln Gln Gly
    1295             1300              1305

Pro Tyr  Gly Ser Ala Ala Ala  Ala Ala Gly Pro Gly  Ser Gly Gln
    1310             1315              1320

Tyr Gly  Gln Gly Pro Tyr Gly  Pro Gly Ala Ser Gly  Pro Gly Gln
    1325             1330              1335
```

```
Tyr Gly  Pro Gly Gln Gln Gly  Pro Ser Ala Ser Ala  Ala Ala Ala
    1340                 1345                 1350

Ala Gly  Ser Gly Gln Gln Gly  Pro Gly Gln Tyr Gly  Pro Tyr Ala
    1355                 1360                 1365

Ser Ala  Ala Ala Ala Ala Gly  Gln Tyr Gly Ser Gly  Pro Gly Gln
    1370                 1375                 1380

Gln Gly  Pro Tyr Gly Pro Gly  Gln Ser Gly Ser Gly  Gln Gln Gly
    1385                 1390                 1395

Pro Gly  Gln Gln Gly Pro Tyr  Ala Ser Ala Ala Ala  Ala Ala Gly
    1400                 1405                 1410

Pro Gly  Gln Gln Gly Pro Tyr  Gly Pro Gly Ser Ser  Ala Ala Ala
    1415                 1420                 1425

Ala Ala  Gly Gln Tyr Gly Tyr  Gly Pro Gly Gln Gln  Gly Pro Tyr
    1430                 1435                 1440

Gly Pro  Gly Ala Ser Gly Gln  Asn Gly Pro Gly Ser  Gly Gln Tyr
    1445                 1450                 1455

Gly Pro  Gly Gln Gln Gly Pro  Gly Gln Ser Ala Ala  Ala Ala Ala
    1460                 1465                 1470

Gly Pro  Gly Gln Gln Gly Pro  Tyr Gly Pro Gly Ala  Ser Ala Ala
    1475                 1480                 1485

Ala Ala  Ala Gly Gln Tyr Gly  Pro Gly Gln Gln Gly  Pro Gly Gln
    1490                 1495                 1500

Tyr Gly  Pro Gly Ser Ser Gly  Pro Gly Gln Gln Gly  Pro Tyr Gly
    1505                 1510                 1515

Pro Gly  Ser Ser Ala Ala Ala  Ala Ala Gly Gln Tyr  Gly Pro Gly
    1520                 1525                 1530

Gln Gln  Gly Pro Tyr Gly Pro  Gly Gln Ser Ala Ala  Ala Ala Ala
    1535                 1540                 1545

Gly Gln  Tyr Gln Gln Gly Pro  Gly Gln Gln Gly Pro  Tyr Gly Pro
    1550                 1555                 1560

Gly Ala  Ser Gly Pro Gly Gln  Gln Gly Pro Tyr Gly  Pro Gly Ala
```

```
             1565                    1570                      1575


        Ser Ala   Ala Ala Ala Ala Gly   Pro Gly Gln Tyr Gly   Pro Gly Gln
             1580                    1585                      1590


        Gln Gly   Pro Ser Ala Ser Ala   Ala Ala Ala Ala Gly   Gln Tyr Gly
             1595                    1600                      1605


        Ser Gly   Pro Gly Gln Tyr Gly   Pro Tyr Gly Pro Gly   Gln Ser Gly
             1610                    1615                      1620


        Pro Gly   Ser Gly Gln Gln Gly   Gln Gly Pro Tyr Gly   Pro Gly Ala
             1625                    1630                      1635


        Ser Ala   Ala Ala Ala Ala Gly   Gln Tyr Gly Pro Gly   Gln Gln Gly
             1640                    1645                      1650


        Pro Tyr   Gly Pro Gly Gln Ser   Ala Ala Ala Ala Ala   Gly Pro Gly
             1655                    1660                      1665


        Ser Gly   Gln Tyr Gly Pro Gly   Ala Ser Gly Gln Asn   Gly Pro Gly
             1670                    1675                      1680


        Ser Gly   Gln Tyr Gly Pro Gly   Gln Gln Gly Pro Gly   Gln Ser Ala
             1685                    1690                      1695


        Ala Ala   Ala Ala Gly Gln Tyr   Gln Gln Gly Pro Gly   Gln Gln Gly
             1700                    1705                      1710


        Pro Tyr   Gly Pro Gly Ala Ser   Ala Ala Ala Ala Ala   Gly Gln Tyr
             1715                    1720                      1725


        Gly Ser   Gly Pro Gly Gln Gln   Gly Pro Tyr Gly Pro   Gly Gln Ser
             1730                    1735                      1740


        Gly Ser   Gly Gln Gln Gly Pro   Gly Gln Gln Gly Pro   Tyr Ala Ser
             1745                    1750                      1755


        Ala Ala   Ala Ala Ala Gly Pro   Gly Ser Gly Gln Gln   Gly Pro Gly
             1760                    1765                      1770


        Ala Ser   Gly Gln Gln Gly Pro   Tyr Gly Pro Gly Ala   Ser Ala Ala
             1775                    1780                      1785


        Ala Ala   Ala Gly Gln Asn Gly   Pro Gly Ser Gly Gln   Gln Gly Pro
             1790                    1795                      1800
```

Gly Gln Ser Gly Gln Tyr Gly Pro Gly Gln Gln Gly Pro Gly Gln
1805 1810 1815

Gln Gly Pro Gly Ser Ser Ala Ala Ala Ala Ala Gly Pro Gly Gln
1820 1825 1830

Tyr Gly Pro Gly Gln Gln Gly Pro Ser Ala Ser Ala Ala Ala Ala
1835 1840 1845

Ala Gly Pro Gly Ser Gly Gln Gln Gly Pro Gly Ala Ser Gly Gln
1850 1855 1860

Tyr Gly Pro Gly Gln Gln Gly Pro Gly Gln Gln Gly Pro Gly Ser
1865 1870 1875

Ser Ala Ala Ala Ala Ala Gly Gln Tyr Gly Ser Gly Pro Gly Gln
1880 1885 1890

Gln Gly Pro Tyr Gly Ser Ala Ala Ala Ala Ala Gly Pro Gly Ser
1895 1900 1905

Gly Gln Tyr Gly Gln Gly Pro Tyr Gly Pro Gly Ala Ser Gly Pro
1910 1915 1920

Gly Gln Tyr Gly Pro Gly Gln Gln Gly Pro Ser Ala Ser Ala Ala
1925 1930 1935

Ala Ala Ala Gly Ser Gly Gln Gln Gly Pro Gly Gln Tyr Gly Pro
1940 1945 1950

Tyr Ala Ser Ala Ala Ala Ala Ala Gly Gln Tyr Gly Ser Gly Pro
1955 1960 1965

Gly Gln Gln Gly Pro Tyr Gly Pro Gly Gln Ser Gly Ser Gly Gln
1970 1975 1980

Gln Gly Pro Gly Gln Gln Gly Pro Tyr Ala Ser Ala Ala Ala Ala
1985 1990 1995

Ala Gly Pro Gly Gln Gln Gly Pro Tyr Gly Pro Gly Ser Ser Ala
2000 2005 2010

Ala Ala Ala Ala Gly Gln Tyr Gly Tyr Gly Pro Gly Gln Gln Gly
2015 2020 2025

Pro Tyr Gly Pro Gly Ala Ser Gly Gln Asn Gly Pro Gly Ser Gly
2030 2035 2040

EP 3 632 988 A1

```
Gln Tyr  Gly Pro Gly Gln Gln  Gly Pro Gly Gln Ser  Ala Ala Ala
    2045                2050             2055

Ala Ala  Gly Pro Gly Gln Gln  Gly Pro Tyr Gly Pro  Gly Ala Ser
    2060                2065             2070

Ala Ala  Ala Ala Ala Gly Gln  Tyr Gly Pro Gly Gln  Gln Gly Pro
    2075                2080             2085

Gly Gln  Tyr Gly Pro Gly Ser  Ser Gly Pro Gly Gln  Gln Gly Pro
    2090                2095             2100

Tyr Gly  Pro Gly Ser Ser Ala  Ala Ala Ala Ala Gly  Gln Tyr Gly
    2105                2110             2115

Pro Gly  Gln Gln Gly Pro Tyr  Gly Pro Gly Gln Ser  Ala Ala Ala
    2120                2125             2130

Ala Ala  Gly Gln Tyr Gln Gln  Gly Pro Gly Gln Gln  Gly Pro Tyr
    2135                2140             2145

Gly Pro  Gly Ala Ser Gly Pro  Gly Gln Gln Gly Pro  Tyr Gly Pro
    2150                2155             2160

Gly Ala  Ser Ala Ala Ala Ala  Ala Gly Pro Gly Gln  Tyr Gly Pro
    2165                2170             2175

Gly Gln  Gln Gly Pro Ser Ala  Ser Ala Ala Ala Ala  Ala Gly Gln
    2180                2185             2190

Tyr Gly  Ser Gly Pro Gly Gln  Tyr Gly Pro Tyr Gly  Pro Gly Gln
    2195                2200             2205

Ser Gly  Pro Gly Ser Gly Gln  Gln Gly Gln Gly Pro  Tyr Gly Pro
    2210                2215             2220

Gly Ala  Ser Ala Ala Ala Ala  Ala Gly Gln Tyr Gly  Pro Gly Gln
    2225                2230             2235

Gln Gly  Pro Tyr Gly Pro Gly  Gln Ser Ala Ala Ala  Ala Ala Gly
    2240                2245             2250

Pro Gly  Ser Gly Gln Tyr Gly  Pro Gly Ala Ser Gly  Gln Asn Gly
    2255                2260             2265

Pro Gly  Ser Gly Gln Tyr Gly  Pro Gly Gln Gln Gly  Pro Gly Gln
    2270                2275             2280
```

39

```
Ser Ala   Ala Ala Ala Ala Gly   Gln Tyr Gln Gln Gly   Pro Gly Gln
    2285                  2290                  2295

Gln Gly   Pro Tyr Gly Pro Gly   Ala Ser Ala Ala Ala   Ala Ala Gly
    2300                  2305                  2310

Gln Tyr   Gly Ser Gly Pro Gly   Gln Gln Gly Pro Tyr   Gly Pro Gly
    2315                  2320                  2325

Gln Ser   Gly Ser Gly Gln Gln   Gly Pro Gly Gln Gln   Gly Pro Tyr
    2330                  2335                  2340

Ala Ser   Ala Ala Ala Ala Ala   Gly Pro Gly Ser Gly   Gln Gln Gly
    2345                  2350                  2355

Ser Ser   Val Asp Lys Leu Ala   Ala Ala Leu Glu His   His His His
    2360                  2365                  2370

His His
    2375
```